# EUROPÄISCHE PATENTSCHRIFT

(11) **EP 1 401 466 B1**
(45) Veröffentlichungstag und Bekanntmachung des Hinweises auf die Patenterteilung: **04.05.2005**
(21) Anmeldenummer: 02760209.3
(22) Anmeldetag: 01.07.2002
(51) Int. Cl.: A61K 35/78, A61P 17/02

(54) **FETT(ÖL)HALTIGES MITTEL, ENTHALTEND ZWIEBELEXTRAKT, SEINE HERSTELLUNG UND SEINE VERWENDUNG ZUR PFLEGE, VORBEUGUNG ODER BEHANDLUNG VON GESCHÄDIGTEM HAUTGEWEBE, INSBESONDERE VON NARBEN**
AGENT CONTAINING FAT (OIL), WHICH CONTAINS ONION EXTRACT, THE PRODUCTION AND USE THEREOF FOR CARING, PREVENTING OR TREATING DAMAGED SKIN TISSUE, ESPECIALLY SCARRED TISSUE
AGENT CONTENANT DE LA GRAISSE(DE L'HUILE) ET UN EXTRAIT D'OIGNON, SA PRODUCTION ET SON UTILISATION POUR SOIGNER, PREVENIR OU TRAITER DES TISSUS CUTANES ENDOMMAGES, NOTAMMENT DES CICATRICES

(30) Priorität: 03.07.2001 DE 10132003
(43) Veröffentlichungstag der Anmeldung: 31.03.2004
(73) Patentinhaber: Merz Pharma GmbH & Co. KGaA, 60318 Frankfurt (DE)
(72) Erfinder: PASPALEEVA-KÜHN, Valentina, 60323 Frankfurt / Main (DE); BEUTLER, Rolf, D., 64739 Höchst/Hummetroth (DE); SCHATSCHNEIDER, Simone, 65207 Wiesbaden (DE); HEBERER, Martina, 63073 Offenbach (DE)
(74) Vertreter: Müller, Claudia, Dr.
(86) Internationale Anmeldenummer: PCT/EP2002/007216
(87) Internationale Veröffentlichungsnummer: WO 2003/004043

(56) Entgegenhaltungen:
- EP-A- 0 273 407
- EP-A- 0 818 203
- WO-A-98/50054
- WO-A-02/055049
- FR-A- 2 681 531
- US-A- 5 885 581
- ROSSI A. ET AL: "[Treatment of keloid and hypertrophic scar with Kelimed.RTM. ointment]. IL TRATTAMENTO DEI CHELOIDI E DELLE CICATRICI IPERTROFICHE CON KELIMED.RTM. UNGUENTO." CHRONICA DERMATOLOGICA, Bd. 6, Nr. 2, 1996, Seiten 275-281, XP009000663
- "Contractubex-das Narbenspezificum" MERZ HOMEPAGE, [Online] XP002220461 Gefunden im Internet: <URL:http://www.merz.de/wwwmerzde/patiente ninformation/narben/contractubex__das_narb enspezifikum.HTM> [gefunden am 2002-11-11]
- WILLITAL G.H. ET AL: "Efficacy of contractubex.RTM. gel in the treatment of fresh scars after thoracic surgery in children and adolescents." INTERNATIONAL JOURNAL OF CLINICAL PHARMACOLOGY RESEARCH, Bd. 14, Nr. 5-6, 1994, Seiten 193-202, XP009000661
- JACKSON B A ET AL: "Pilot study evaluating topical onion extract as treatment for postsurgical scars." DERMATOLOGIC SURGERY: OFFICIAL PUBLICATION FOR AMERICAN SOCIETY FOR DERMATOLOGIC SURGERY [ET AL.]. UNITED STATES APR 1999, Bd. 25, Nr. 4, April 1999 (1999-04), Seiten 267-269, XP002220462 ISSN: 1076-0512

## Beschreibung

Die vorliegende Erfindung betrifft ein neues fett(öl)haltiges Mittel, enthaltend Zwiebelextrakt, sowie seine Herstellung und seine Verwendung zur Herstellung von Zusammen Setzungen zur Pflege, Vorbeugung oder Behandlung von geschädigtem Hautgewebe, wie z.B. nach Operationen, Biopsien, Schnittwunden, Verbrennungen und sonstigen Unfällen, sowie insbesondere von Narben, Schwangerschaftsstreifen , degenerativen Hautveränderungen u.ä.. Das Mittel ist dadurch gekennzeichnet dass es eine Ölgrundlage aufweist und insofern z.B. auf Creme-, Lotion-, Fluid-, Massageöl- oder Balsamgrundlage hergestellt werden kann . Es ist daher fett(öl)haltig. Überraschenderweise konnte der Zwiebelextrakt, welcher insbesondere Wasser, Alkohol, oder auch Wasser-Alkohol - haltig ist, in eine solche ölhaltige Grundlage eingebaut werden, ohne dass seine Wirksamkeit beeinträchtigt wird oder eine Phasentrennung auftritt. Die Wirksamkeit konnte in medizinischen Anwendungsbeobachtungen nachgewiesen werden.

Mittel zur Behandlung von geschädigtem Hautgewebe, insbesondere von Narben sind bekannt. Insbesondere werden hierfür gelartige Produkte eingesetzt. Die US - A - 5,885,581 beschreibt ein solches gelartiges Produkt, welches 20 - 30 Gewichtsprozent Polyethylenglykol 200, 0,005- 0,03 Gewichtsprozent Konservierungsmittel, 0,05- 0,2 Gewichtsprozent Sorbinsäure, 0,5- 2 Gewichtsprozent Allantoin, 1- 3 Gewichtsprozent Xanthan und wahlweise Parfümstoffe umfasst und welches gekennzeichnet ist durch 5- 15 Gewichtsprozent eines flüssigen Zwiebelextraktes (Extrakt Allium Cepa), auf der Basis eines wässrigen Trägers in einer Menge von etwa 55- 65 Gewichtsprozent. Das Produkt stellt somit ein fett(öl)- freies Gel dar und wird äußerlich auf geschädigtes Hautgewebe, insbesondere vernarbtes Gewebe aufgetragen. Das Produkt ist ferner gekennzeichnet durch einen pH- Wert von 4,5- 5,5 und eine Partikelgröße von weniger als 50µm.

Weiterhin ist ein Spezialpflaster gegen Narben bekannt, welches unter dem Namen "Hansaplast® Narbenreduktion" ohne Wirkstoffe zu einer besseren Heilung verletzter Haut führen soll. Dabei werden entsprechende Pflasterpads etwa 8- 10 Wochen auf die Haut gelegt und fördern durch das gesteigerte Temperatumiveau angeblich den Regenerationsprozeß.
Unter dem Produkt Kelofibrase® ist eine Narbencreme bekannt, welche als Wirkstoffe Harnstoff sowie Heparin- Natrium (60 000 I.E.) und Kampfer neben üblichen Öl/Emulgator Komponenten enthält. Dieses Produkt soll zur Narbenbehandlung, bei Narbenkontrakturen und bei Keloiden einsetzbar sein. Dabei wirkt Heparin- Natrium bekanntlich als blutverdünnendes Mittel.
Unter dem Namen Hirudoid®forte ist ein Gel bekannt, welches als Wirkstoff Mucopolysaccharidpolyschwefelsäureester (445mg, entsprechend 40 000 Einheiten in 100g Salbe) enthält. Weitere Inhaltsstoffe sind die für die Gelherstellung erforderlichen Komponenten wie Isopropylalkohol, Polyacrylsäure, Propylenglycol und Wasser. Mucopolysaccharidpolyschwefelsäureester haben im allgemeinen eine heparinoide Wirkung und entsprechen daher dem zuvor genannten Kelofibrase®-Produkt. Neben dem Gel ist auch eine Hirudoid®forte Salbe bekannt, die neben den oben bekannten Wirkstoffen ein Gemisch aus Mono- und Diglyceriden höherer Fettsäuren und mittelkettige Triglyceride etc. sowie Isopropylalkohol, Imid urea, Phenoxyethanol und Wasser aufweist. Derartige Produkte sind anwendbar zur Behandlung bei Phlebopathien, oberflächennahen Phlebitiden, Hämatomen und zur Auflockerung von harten Narben. Das Produkt darf nicht auf verletzte Haut aufgetragen werden.
Unter dem Namen Hylaform® ist ein Gelimplantat bekannt, welches quervemetzte Hyaluronsäure enthält, die in einer wässrigen kochsalzhaltigen Lösung zwecks Injektion vorhanden ist. Mit einem solchen Mittel sollen Hautdeformationen behandelbar sein. Akute oder chronische Hautkrankheiten im betroffenen Korrekturbereich dürfen jedoch nicht vorliegen.
Unter dem Namen Linoladiol® N ist eine hydrophile O/W- Creme bekannt, welche in der Cremegrundlage als Wirkstoff Estradiol enthält. Mittels dieses hormonhaltigen Präparates sind neben gynäkologischen Applikationen auch dermatologische Anwendungen, wie Verbrennungen, Narbennachbehandlung, Hautatrophie, periorale Dermatitis und Ekzeme im akuten und subakuten Stadium angegeben. Hierbei sind allerdings die für hormonhaltige Produkte bekannten Anwendungbeschränkungen wie Nebenwirkungen bzw.
Wechselwirkungen erheblich zu beachten.
Unter dem Produktnamen PC 30 V ist ein Liquidum beschrieben, welches Roßkastaniensamentrockenextrakt sowie Kamillenblütentrockenextrakt in 1,3-Butandiol, Dexpanthenol, Allantoin und Geruchsstoffe enthält. Dieses Mittel soll einsetzbar sein bei der Behandlung von Hautschädigungen wie Wundscheurn empfindlicher Druckstellen und Narben durch orthopädische Apparate, sowie Wundliegen. Eine Indikation bezüglich Narben durch Operationen oder sonstige
Hautschädigungen ist hier nicht angegeben.
Die Salbe Striatridin® enthält neben der Salbengrundlage als Wirkstoff alkylverzweigte Fettsäureester des Octadecylalkohols, Aminosäuresol sowie Ethylnicotinat. Dieses Produkt ist zur Narbenbehandlung, bei Schwangerschaftsstreifen sowie bei schlaffer, funktionsgeschädigter Haut einsetzbar.
Die DE- A 196 28 284 beschreibt den Einsatz von Bärlauch zur Behandlung von Schuppenflechte.
Die DE- A 37 23 248 betrifft die Verwendung von Thiosulfinsäurederivaten zur Behandlung von Entzündungen. Diese können u.a. durch Extraktion von Zwiebeln und nachfolgende Chromatographie gewonnen werden. Zwiebelextrakt selbst wird hier nicht eingesetzt.
EP- B 429 080 betrifft ein Herstellungsverfahren für S- Allylcystein enthaltende Produkte, wobei z. B. wässrige Knoblauch -Extrakte mit Cystein versetzt werden, wodurch S-Allylcystein entsteht.
EP- B 364 442 betrifft einen Ölextrakt aus mindestens 3 verschieden Kräutern, ausgewählt aus Wolfsmilch, Veronica, Schafgarbe, Erdrauch, Knoblauch, Nessel und Ringelblume. Diese Kombination wird als Öl, z.B. mit Paraffin gegen Schuppenflechte angewendet.
EP-B EP 201 956 betrifft die Extraktion und chromatographische Fraktionierung z.B. von Tabak, Algen, Knoblauch, wobei die erhaltenen spezifischen Substanzen als antioxidative Substanzen in Kosmetika eingesetzt werden sollen.
EP-A-0273407, WO98/50054 und US-A-5885581 beschreiben Hydrogele, die Zwiebelextrakte enthalten US- A 6,200,570 betrifft Zusammensetzungen, enthaltend Knoblauchextrakt und mindestens einen weiteren Pflanzenextrakt wie Aloe Vera sowie entzündungshemmende Mittel wie Diclofenac mit anti- allergischer, analgesischer Wirkung.
JP-A 2000327535 beschreibt ein Haartonikum, welches z.B. Allium Schoenoprasum und/oder andere Pflanzenextrakte aufweist.
JP-A 09194334 betrifft ein gegen Haarausfall wirksames Haartonikum, das z. B. Allium Sativum und/oder andere Pflanzenextrakte aufweist.
JP-A 08012570 und JP-A 0317413 betreffen Pflanzenextrakte wie Allium sativum oder Allium victorialis enthaltende anti- allergene oder Anti- Schuppenmittel.
Aus dem vorgenannten Stand der Technik ergibt sich, dass die zur Narbenbehandlung einsetzbaren Produkte entweder als Gel vorliegen oder, wenn sie nicht als Gel vorliegen, Wirkstoffe enthalten, welche erhebliche Nebenwirkungen mit sich bringen können. So ist z.B. das Estradiol enthaltende Produkt anwendungsbeschränkt im Hinblick auf den Hormongehalt. Mucopolysaccharidpolyschwefelsäureester mit ihrer heparinoiden Wirkung können Überempfindlichkeitsreaktionen auslösen und dürfen auf keinen Fall auf eine verletzte Haut aufgetragen werden. Das oben genannte Hylaform®-Hyaluronsäure enthaltende Produkt ist ein Implantat, welches nur zur mechanischen Korrektur von Hautdeformationen geeignet sein kann und insofern keine dauerhafte Einwirkung auf das Hautgewebe selbst mit sich bringt. Das Liquidum PC 30 V ist lediglich zur Druckstellennarbenbehandlung geringen Umfangs, wie z.B. durch orthopädische Apparate verursacht, einsetzbar und insofern zur dauerhaften Veränderung geschädigten Hautgewebes durch Operationen, Verletzungen, Verbrennungen etc. nicht beschrieben.

Es besteht daher ein Bedürfnis nach einem Produkt, welches die oben genannten Nebenwirkungen nicht aufweist. Ferner sollte es nicht in Gelform vorliegen, da fett(öl)- freie Gele bekanntlich eine eher austrocknende Wirkung auf die Haut ausüben können.

Aufgabe vorliegender Erfindung ist es daher, ein Produkt bereitzustellen, welches einen gut verträglichen Wirkstoff aufweist und wobei dieser in eine Emulsionsgrundlage eingearbeitet ist, wodurch die Haut nicht ausgetrocknet wird und darüber hinaus noch ein positiver, pflegender, insbesondere elastizitätsfördernder Hautpflegeeffekt erzielt werden kann.

Diese Aufgabe wird erfindungsgemäß gelöst durch ein Mittel zur topischen Anwendung, welches als Wirkstoff einen Zwiebelextrakt aufweist und neben den üblichen Zusatzstoffen, ausgewählt aus Konsistenzgebern, Farbstoffen, Antioxidantien, Parfümstoffen, Feuchthaltemittel, Konservierungsmittel, Stabilisatoren, Zusatzwirkstoffen, eine Fett(Öl-)phase aufweist.
Bevorzugt können zusätzlich eine Wasserphase und entsprechende Emulgatoren, ausgewählt aus der Gruppe aus O/W- und/oder W/O Emulgatoren oder Mischungen hiervon oder mit geeigneten Co-Emulgatoren enthalten sein.

Die Mittel können insgesamt 5-99% Fett(öl)phase, 0,1-35% Zusatzstoffe, 1-20% Zwiebelextrakt und die übrigen Stoffe, sofern vorhanden, in den unten angegebenen Mengen aufweisen.
Besonders geeignet sind Mittel, welche zusammengesetzt sind aus 70-99, ganz besonders 80-98% Fett(öl)phase, 0,1-20%, insbesondere 0,1-10%, Zusatzstoffe und 1-20%, bevorzugt 1-10% und ganz besonders 2-5%, Zwiebelextrakt enthalten, wobei bevorzugt als Rest Lösungsmittel vorliegen können, wie z. B. Alkohole ( Ethanol, Isopropanol ) z.B. in Mengen von bis zu 20%.
Das erfindungsgemäße Mittel ist ferner bevorzugt zusammengesetzt aus einer Fett(öl)phase in einer Menge von 5- 70%, 0,1- 15% Emulgator (O/W; W/O); Mischungen hiervon oder mit Co-Emulgatoren, 0,1- 35% Zusatzstoffen, 1- 20% Zwiebelextrakt und der Rest Wasser oder ein Wasser/Alkohol- z. B. Ethanol, Isopropanol, -Mischungen, z.B. bis 20%.
Insbesondere sind als Fett(öl) phase 5-60%, bevorzugt 5-40% und ganz besonders 5-25% geeignet.
Der Zwiebelextrakt ist insbesondere ein Wasser - oder Alkohol - oder Wasser-Alkohol - aufweisender Zwiebelextrakt.

Mit einer solchen Zusammensetzung ist es überraschenderweise möglich, den Zwiebelextrakt so in eine Fett(öl)haltige Phase einzuarbeiten, dass hierbei eine gleichförmige Verteilung des Extraktes stabil vorliegt und darüber hinaus mittels dieser Grundlage ein Austrocknen der behandelten Haut nicht stattfinden kann und somit auch ein pflegender, elastizitätsfördernder Effekt erzielt wird, so dass die Behandlung der geschädigten Hautteile sowohl hinsichtlich der Vernarbung bzw. Erschlaffung als auch bezüglich der Hautweichheit positiv beeinflusst bzw. verhindert werden kann.

Besonders geeignet sind im erfindungsgemäßen Mittel zwischen 1- 15%, 2-15%, insbesondere 5-15% des Zwiebelextraktes, bezogen auf die Gesamtmenge , vorzugsweise 5- 10, insbesondere 8- 10% und ganz besonders bevorzugt 2-4% oder 10%.
Die Zusatzstoffe liegen bevorzugt bei 0,1- 30, insbesondere 0,1-25% je nach Anwendungszweck vor.
Die Menge an Emulgatoren beträgt insbesondere 0,1-10%,insbesondere 1-10%, bevorzugt 1-8% und ganz besonders 1-5%.
Der Zwiebelextrakt ist insbesondere ein wässrig - ethanolischer Extrakt . Er weist bevorzugt Wasser und 10-15% Ethanol auf.
Es kann auch von Vorteil sein, einen alkoholischen Zwiebelextrakt einzusetzen. Dieser kann neben Alkohol vorzugsweise auch 10-80%, insbesondere 20-60% eines Lösungsmittels, ausgewählt aus Triglyceriden, Kohlenwasserstoffen und Fettsäureestern aufweisen.
Die Mengenangaben beziehen sich auf Gewichts %, sofern nicht anders angegeben.

Die Fett(öl)phase ist bevorzugt ausgewählt aus Kohlenwasserstoffen, Fettalkoholen,-ethem und -estern, (Poly)olfettsäureestem, Triglyceriden, natürlichen Ölen, natürlichen Fetten, Wachsen ,Silikonölen, Silikonwachsen oder Mischungen hiervon. Insbesondere sind hier flüssige Paraffine, Milchsäureester, Fettalkoholether, Nachtkerzenöl, Silikonöl oder Mischungen hiervon bevorzugt.
Der W/O-Emulgator weist vorteilhafter weise einen HLB-Wert von 1-9, insbesondere 1-8, bevorzugt 2-7 und ganz besonders bevorzugt 3-6 und der O/W- Emulgator einen HLB- Wert von 9-18, bevorzugt 9-15 und insbesondere 9-13, auf oder ist ein ionischer O/W- Emulgator.

Als W/O-Emulgatoren sind insbesondere bevorzugt Sorbitan- Derivate, Polyethoxylierte Fettsäuren/Alkohole/Ester/, Triglyceride, (Poly)glycerylderivate, Polyolester, Glucose-Derivate, Pentaerythrit-Derivate, Alkylphenole, (Block)Polymere, Fettsäuresalze, Siloxane oder Mischungen hiervon und hierunter ganz besonders Abil® EM 90, Arlacel ®582 und Magnesiumstearat oder Mischungen hiervon aufweist.
Es können hier auch Co- Emulgatoren vorhanden sein, wie z.B. Arlatone®T(V).
Als O/W-Emulgator eignen sich insbesondere polyoxyethylierte Produkte, nichtionische und ionische Phosphate, ionische einwertige Salze, (Poly)glycerylester, Zuckerester, Sterol-Derivate, Rizinusöl-Derivate, Siloxane oder Gemische hiervon oder Mischungen mit Co-Emulgatoren hiervon. Insbesondere sind hier Tego Care® 450, Eumulgin ®B1 oder Mischungen hiervon und/oder mit Co-Emulgatoren geeignet.

Ganz besonders bevorzugt sind bei Anwesenheit von O/W-Emulgatoren Stabilisatoren, ausgewählt aus Acrylamiden, Acrylaten und Polysacchariden, insbesondere solche, wie sie nachfolgend beschrieben werden.

Es ist ferner bevorzugt, wenn die erfindungsgemäßen Mittel weiterhin als Zusatzstoffe, solche ausgewählt aus Vitaminen, Elektrolyte, wie z. B. Magnesiumsulfat oder Natriumchlorid, Allantoin, D- Panthenol, Hyaluronsäure, Mucopolysacchariden, Farbstoffen, Parfümstoffen, Konservierungsmittel, Feuchthalter aufweisen.
Insbesondere können auch zusätzlich Wachsprodukte sowie oder alternativ als Zusatzstoff weiterhin Lecitine, insbesondere Phosal®50 SA enthalten sein.

Das Mittel kann unterschiedliche Mengen an Wasser, Fettphase, Emulgator, Zusatzstoffe und Wirkstoff (Zwiebelextrakt) enthalten. Hierbei werden dann je nach Anwendungszweck z.B. eine Lotion, ein Fluid, eine Creme oder ein Balsam/Salbe erhalten.
Das Mittel kann somit bevorzugt zusammengesetzt sein aus einer Fett(öl)phase in einer Menge von 5-70% Gewichtsprozent, , insbesondere 5-55%, einem oder mehreren W/O-Emulgatoren, Mischungen hiervon oder mit Co-Emulgatoren in einer Gesamtmenge von 0,5- 15%, insbesondere 0,5-10%, sowie 0,1- 25 %, insbesondere 0,1-22%, an Zusatzstoffen der oben genannten Art, sowie 1- 15, insbesondere 1-10% des oben genannten Zwiebelextraktes und als Rest Wasser oder ein Gemisch von Wasser mit Alkoholen wie z.B. Ethanol , Isopropanol in Mengen bis zu 20%.
Das Mittel kann insbesondere auch zusammengesetzt sein aus 5- 40%, insbesondere 5-30%, Fett(öl)phase, 0,1- 15%, insbesondere 1-10% eines oder mehrerer O/W- Emulgatoren oder Mischungen hiervon oder mit Co-Emulgatoren, 0,1- 32%, insbesondere 0,1-20% Zusatzstoffe und 1- 10% Zwiebelextrakt und als Rest Wasser oder ein Gemisch von Wasser mit Alkoholen wie z.B. Ethanol , Isopropanol in Mengen bis zu 20%.

Auf diese Weise kann das Mittel zur Behandlung von Narben bzw. von geschädigtem und/oder erschlafftem Hautgewebe insbesondere als z.B. Massageöl oder Balsam, Lotion, Fluid oder Creme hergestellt werden. So können bei Verwendung von W/O- Emulgatoren insbesondere Lotionen, und ein Balsam und bei Einsatz von O/W- oder W/O- Emulgatoren insbesondere cremeartige Produkte oder Fluids entstehen. Je nach Einsatzgebiet kann zwischen diesen Produkten ausgewählt werden. Insbesondere geeignet ist eine O/W-Creme der oben beschriebenen Art zur Behandlung z.B. von Aknenarben. Sollen größerflächige Bereiche behandelt werden, empfiehlt sich eine Lotion, die bevorzugt bei der Behandlung oder auch zur vorbeugenden Verhinderung von Schwangerschaftsstreifen oder allgemein erschlaffter Haut einsetzbar ist. Ebenfalls größerflächig einsetzbar ist ein Balsam, der ganz besonders zur Behandlung von Operations- oder Verbrennungsnarben, Schnittwunden geeignet ist. Massageöl ist insbesondere geeignet zur Vorbeugung oder Pflege und Behandlung von größeren Hautflächen wie z.B. bei Schwangerschaftssteifen. Die Darreichungsform ist jedoch nicht auf eine bestimmte Indikation beschränkt und kann varriert werden.

Die einzelnen Inhaltsstoffe werden nachfolgend näher beschrieben:

### I. Zwiebelextrakt

Als Zwiebelextrakt eignet sich insbesondere ein Wasser , Alkohol oder Wasser-Alkohol enthaltender Auszug aus getrockneten Zwiebeln. Die Extraktion kann dabei mit Wasser selbst oder auch mit Alkohol, ggf. unter Zusatz von Lösungsmitteln wie nachstehend genannt, oder einem Gemisch aus Wasser und einem oder mehreren Alkoholen, wie nachfolgend genannt, vorgenommen werden. Die getrockneten Zwiebeln, auch als getrocknete Zwiebelchips bekannt, erhalten aus der Stammpflanze Allium cepa Linne, können dabei zunächst mit dem Extraktionsmittel extrahiert werden, indem man die Droge vorzugsweise in der Wärme, z.B. bei 40-90°C, erschöpfend perkoliert. Das Perkolat kann dann bei geeigneter, insbesondere erhöhter Temperatur über 30°C unter Vakuum zum Spissum- Extrakt (eingedickter Extrakt) eingedampft und anschließend im gewünschten Lösungsmittel zum Wasser-, Alkohol- oder Wasser-Alkoholgemisch - Fluidextrakt aufgelöst werden. Alternativ kann der Fluidextrakt auch ohne zuvoriges Eindampfen durch direktes Umsetzen mit dem gewünschten Lösungsmittel erhalten werden.
Erhalten wird eine rötlich- braune bis braune Flüssigkeit von charakteristischem Geruch. Das Verhältnis von trockener Droge zu Fluidextrakt kann dabei variieren von 0,1:1 bis 10:1, bevorzugt 0,1:1 bis 5:1, insbesondere 0,15:1 bis 4:1. Ganz besonders bevorzugte Verhältnisse sind 0,16:1 oder 4:1.
Besonders bevorzugt sind Fluidextrakte der genannten Art mit Wasser/Alkohol-Gemisch oder nur Alkohol als Lösungsmittel. Im Gemisch ist bevorzugt 5-60%, insbesondere 10-50% und ganz besonders 10-40% oder auch 10-15, vor allem 13%, Alkohol, bevorzugt Ethanol, enthalten.
Der Alkohol ist vorzugsweise ausgewählt aus Ethanol, Isopropanol oder Propanol oder zweiwertigen Alkoholen wie Butylenglykol, Propylenglykol oder Mischungen hiervon. Insbesondere bevorzugt ist Ethanol.
Es kann ferner von Vorteil sein, wenn der Zwiebelextrakt, welcher mit Wasser oder anderen wie z.B. den oben genannten alkoholischen Lösungsmitteln extrahiert wird, zum alkoholischen Fluidextrakt wie beschrieben, aufgearbeitet wird. Insbesondere können hierbei 10-80%, bevorzugt 20-60% des Alkohols durch ein Lösungsmittel, ausgewählt aus den nachfolgend unter Punkt II genannten Stoffen, insbesondere Kohlenwasserstoffe, Triglyceride, besonders mittelkettige, und Fettsäureester oder Mischungen hiervon, ersetzt werden.
Ein erfindungsgemäß verwendbarer Zwiebelextrakt kann beispielsweise wie folgt hergestellt werden:
Die getrockneten Zwiebeln, auch Zwiebelchips genannt, können z.B. mit gereinigtem Wasser z.B. im Verhältnis von Droge zu Auszugsmittel (Wasser) von 1:16 extrahiert werden. Das Verhältnis von trockener Droge zu nativem Extrakt entspricht dann 1,8:1, bzw. 1,5-2,2:1. Das Verhältnis von trockener Droge zu Fluidextrakt ist dann 0,16:1. Die Droge (z.B. 16kg Chips) wird mit heißem Wasser (80- 90 Grad Celsius) erschöpfend perkoliert. Das Perkolat kann dann bei ca. 55 Grad Celsius unter Vakuum zum Spissum-Extrakt (eingedickter Extrakt) eingedampft werden. Dabei wird kurzzeit erhitzt (z.B. 3 Sekunden bei 141 Grad Celsius). Der erhaltene Spissumextrakt ist dickflüssig und wird in Wasser, Alkohol oder im Wasser- Alkoholgemisch zum Fluidextrakt z.B. o.g. Verhältnis aufgelöst. Erhalten wird eine rötlich- braune bis braune Flüssigkeit von charakteristischem Geruch. Dieser Extrakt ist mit Wasser in jedem Verhältnis mischbar, weist eine relative Dichte bei 20 Grad Celsius von 1,00- 1,03 g/ml auf. Sofern mit Wasser/Alkohol, insbesondere Ethanol gearbeitet wurde, können z.B. etwa 13- 20% (V/V) Alkohol (z.B. Ethanol) vorliegen. Der Trockenrückstand (nach 2 Stunden bei 105 Grad Celsius) beträgt mindestens 7,0 % (m/m). Hinsichtlich der mikrobio-logischen Reinheit entspricht das Produkt den Anforderungen der Kategorie 3 des DAB 10 .
Die charakteristischen Inhaltsstoffe der Zwiebeln sind Derivate von schwefelhaltigen Aminosäuren . Daneben liegen noch Eiweiß, Fett und Kohlenhydrate vor.

Das wie oben beschrieben hergestellte Produkt ist beispielsweise erhältlich von Fa. Finzelberg Extrakte und beschrieben in der o. g. US-A-5885581.
Dieser wie oben beschrieben gewonnene Extrakt ist im Narbengel, gemäß der US Patentschrift 5885581 eingesetzt. Im Unterschied zur Gelzusammensetzung war es jedoch überraschend, diesen Extrakt in eine ölhaltige oder Öl-Wasserhaltige Grundlage einzuarbeiten, wobei gleichzeitig eine vollständige und homogene Verteilung der Wirkstoffe und auch insbesondere ihr Intaktbleiben gewährleistet bleibt, zumal, wenn der Anteil an dieser Extraktphase, insbesondere in wässriger, alkoholischer, wässrig/alkoholischer Form, sehr hoch ist.
Bevorzugt wird also ein Wasser- Alkohol- Zwiebelextrakt in den erfindungsgemäßen Mitteln eingesetzt, insbesondere ein wie oben beschrieben Wasser-Ethanol Extrakt, vorzugsweise mit 10-40, insbesondere 13-20%, insbesondere 10-15% Ethanolanteil.
Wie erwähnt kann der Zwiebelextrakt statt Ethanol auch andere Lösungsmittel, insbesondere Glykol, Butylenglykol, Propylenglykol, aufweisen.
Ferner bevorzugt ist auch ein Alkohol-, bevorzugt -Ethanol- haltiger Extrakt, welcher insbesondere darüber hinaus wie erwähnt , noch weitere Lösungsmittel, z.B. 10-80%, insbesondere 20-60%, wie die unter Punkt **II** genannten aufweisen kann. Bevorzugt sind hier Kohlenwasserstoffe, Triglyceride z. B. mittelkettige, Fettsäureester.
Ferner sind Mischungen aus einem Wasser-Alkohol, insbesondere Ethanol-Zwiebelextrakt, z.B. mit 10-40% Ethanol wie oben beschrieben und einem alkoholischen, insbesondere ethanolischen Zwiebelextrakt mit 10-80%, insbesondere 20-60%,Lösungsmittel wie die unter Punkt **II** genannten , insbesondere hier Kohlenwasserstoffe, Triglyceride z. B. mittelkettige, Fettsäureester geeignet. Das Verhältnis von Wasser/Alkohol- und Alkohol/Lösungsmittel-Zwiebelextrakt kann insbesondere 3:1 bis 1:3, bevorzugt 1:1 betragen.

### II.Fett(Öl)phase

Für die Fett(Öl-)phase können hierfür übliche Öl- Komponenten eingesetzt werden. Hierzu gehören:
Übliche, vorzugsweise flüssige Lipide, diese können einzeln oder im Gemisch vorliegen.
Insbesondere sind die folgenden Gruppen und Beispiele hierfür geeignet:
1. Kohlenwasserstoffe
   Kohlenwasserstoffe wie Squalen, Squalan, insbesondere auch flüssige Paraffine (Paraffinum Perliquidum), Isoparaffine, Dioctylcyclohexane (Cetiol® S), Isohexadecane (Arlamol® HD);
2.Fettalkohole wie Oleylalkohol, Octyldodecanol (Eutanol® G);
3.Fettsäureester, z.B. Isopropylfettsäureester (Palmitat, Myristat, Isostearat, Oleat), Decyl Oleate (Cetiol® V), Hexyl Laurate, C 12 - 15 Alkyl Benzoate (Finsolv® TN), Dicaprylyl Carbonate (Cetiol® CC), Diester wie Dibutyladipate (Cetiol®B), Propylenglykol Dipelargonate, verzweigte Fettsäureester wie PCLliquid® (Cetearyl Octanoate) oder Gemische wie Cetiol® PGL (Hexyldecanol und Hexyldecyl Laurate);
4.Fettalkoholether wie Dicaprylyl Ether (Cetiol® OE) oder Fettalkoholester wie Milchsäurester wie C12-13 Alkyl Lactate (Cosmacol® ELI);
5.Polyolfettsäureester wie Cetiol® HE (PEG-7 Glyceryl Cocoate);
6.Triglyceride, insbesondere mittelkettige (Neutralöle) wie Caprylic/Capric Triglyceride (Miglyol® 810, 812) sowie deren Polyolester wie Propylene Glycol Dicaprylate/ Dicaprate (Miglyol® 840);
7.Natürliche Fette und Öle wie Sonnenblumen-, Soja-, Pfirsichkern-, Aprikosenkern-, Traubenkern-, Ricinus-, Erdnuß-, Mandel-, Nerz-, Weizenkeim-, Avocadoöl, Nachtkerzenöl;
8.Wachse, wie natürliche flüssige Wachse z.B. Jojobaöl oder dessen Substitut Oleyl Erucate (Cetiol® J 600) oder synthetische Wachse wie die nachfolgend unter "Konsistenzgeber" beschriebenen. 9.Silikonöle und -wachse, z.B. Polydimethylsiloxane wie Dow Corning Fluid® 200 (Dimethicone), Cyclomethylsiloxane wie Dow Corning Fluid® 345 (Cyclomethicone), Phenylmethylpolysiloxane wie Phenyl Dimethicone (Abil® AV 8853) oder Alkyl-Polymethylsiloxan-Copolymere wie Cetyl Dimethicone (Abil® Wax 9801), Stearyl Dimethicone (Abil® Wax 9800), Dialkoxydimethylpolysiloxane wie Stearoxy
   Dimethicone (Abil® Wax 2434), Behenoxy Dimethicone (Abil® Wax 2440)

Besonders bevorzugte Öl-Komponenten sind flüssige Paraffine, Fettsäureester wie Isopropylpalmitat oder -myristat, Fettalkoholether wie Dicaprylyl Ether (Cetiol®OE) sowie die genannten natürlichen Fette und Öle, insbesondere Avokado-, Soja-, Pfirsichkern-, Aprikosenkernöl und ganz besonders Nachtkerzenöl, insbesondere Mischungen hiervon, Silikonöle der vorbeschriebenen Art sowie Milchsäureester z.B. Cosmacol® ELI sowie Mischungen hiervon mit den vorgenannten Komponenten.
Insbesondere bevorzugt werden die genannten flüssigen Paraffine, Milchsäureester, Fettalkoholether, Nachtkerzenöl und Silikonöle, auch in Kombination miteinander, wobei ca. 1- 50% an Einzelkomponente bezogen auf die Gesamtmenge an Öl vorhanden sein können.
Ferner sind auch Silikonwachse besonders geeignet, vor allem auch Kombinationen hiervon und auch mit den vorgenannten Paraffinen, Milchsäureester und Nachtkerzenöl.

### III.Emulgatoren

### A). W/O Emulgatoren

Als W/O- Emulgator/en eignen sich solche mit einem geeigneten HLB- Wert von 1-9, insbesondere1- 8. Besonders bevorzugt sind folgende Produkte:
1.Sorbitan-Derivate
   Sorbitan Ester wie Sorbitan Oleate (Span® 80, HLB = 4,5), Sorbitan Stearate (HLB = 5,0), Sorbitan Sesquioleate (Crill® 43, HLB = 3,7), Sorbitan Isostearate (Crill® 6, HLB = 4,7), Sorbitan Tristearate (Crill® 35, HLB = 2,1);
   sowie Arlacel® 582 (Sorbitan Isostearate, PEG-2 Hydrogenated Castor Oil, Ozokerite, Hydrogenated Castor Oil, HLB = 5).
2.Polyethoxylierte Produkte
   Polyethoxylierte Fettsäuren und -alkohole wie PEG-2 Oleate (HLB = 5,0), PEG-4 -Distearate (HLB = 3,0), PEG-2 Stearate (HLB = 4,4), Ceteareth-3, (Volpo® CS3, HLB = 5,0), Ceteth-2 (Volpo® C2, HLB = 5,3);
   Polyoxyethylen Fettsäureester wie Arlatone® T(V) (PEG-40 Sorbitan Peroleate, HLB = 9);
   Ethoxylierte Triglyceride wie PEG-5 Castor Oil (HLB = 3,9), PEG-6 Diricinoleate (HLB = 5,0), PEG-7 Hydrogenated Castor Oil (Cremophor® WO 7, HLB = 5,0);
3.(Poly)glycerylderivate
   Polyglycerylester wie Polyglyceryl-3 Diisostearate (Lameform® TGI, HLB = 3,5), Polyglyceryl-2 Dipolyhydroxystearate (Dehymuls® PGPH, HLB = 3,5), Diisostearoyl Polyglyceryl-3 Diisostearate (Isolan® PDI, HLB ca. 5), Polyglyceryl-3 Oleate (Isolan® GO 33, HLB ca. 5), Polyglyceryl-3 Dioleate (Cremophor® GO 32, HLB ca. 5), Polyglyceryl-4 Isostearate (Isolan® GI 34, HLB ca. 5);
   Glycerin-Ester wie Glyceryl Ricinoleate (Cithrol® GMR N/E, HLB = 2,7), Glyceryl Laurate (Cithrol® GML N/E, HLB = 4,9), Glyceryl Dioleate S/E (Cithrol® GDO S/E, HLB = 2,9).
4. Polyolester
   Polyol-Ester wie Glycol Oleate S/E (Cithrol® EGMO S/E, HLB = 2,7), Glycol Ricinoleate (Cithrol® EGMR S/E, HLB = 2,0), Glycol Dilaurate S/E (Cithrol®EGDL S/E, HLB = 2,0), Propylene Glycol Ricinoleate (Cithrol® PG MR S/E, HLB = 3,6), Propylenglykol Laurate (Cithrol® PGML N/E, HLB = 2,7);
5.Glucose-Derivate
   Glucose Ester wie Methyl Glucose Dioleate (Isolan® DO, HLB ca. 5), Methyl Glucose Isostearate (Isolan® IS, HLB ca. 5);
6.Pentaerythrit-Derivate
   Pentaerythrit-Fettsäureester, z.B. Pentraerythrityl Monolaurate (HLB = 4,8), Pentaerythrityl Monotallate (HLB = 4,0) oder Mischester, z.B. mit Citronensäure Fettalkoholester wie Dehymuls® E (Dicocoyl Pentaerythrityl Distearyl Citrate, Sorbitan Sesquioleate, Cera Alba, Aluminium Stearate, HLB = 4,0), Dehymuls® F (Dicocoyl Pentaerythrityl Distearyl Citrate, Cera Microcristallina, Glyceryl Oleate, Aluminium Stearate, Propylene Glycol, HLB = 4,0);
7.Alkylphenole
   z.B. Nonoxynol-2 (HLB ca. 4,5);
8.Polymere
   Polymere wie Polyoxypropylen-polyoxyethylen-Blockpolymere (INCI-Name: Poloxamere), z.B. Pluronic® PE 3100 (HLB = 4,5), Pluronic®PE 6100 (HLB = 3,0) oder PEG-30 Dipolyhydroxystearate (Arlacel® P 135, HLB ca. 5,5);
9.Siloxan-Derivate
   Polysiloxan-Copolymere wie Polysiloxan-Polyether-Copolymere, insbesondere Polysiloxan-Polyalkyl-Polyether-Copolymere wie Cetyl Dimethicone Copolyol (Abil® EM 90, HLB = ca. 5), Laurylmethicone Copolyol (Dow Corning® Q2-5200, HLB ca. 4) oder deren Gemische wie Abil® WE 09 (Cetyl Dimethicone Copolyol Polyglyceryl-4-Isostearate, Hexyl Laurate, HLB ca. 5).
10.Fettsäure-Salze
   mehrwertige Salze wie Magnesium-, Aluminium-, oder Zinkstearat, wobei Magnesiumstearat bevorzugt ist.

Insbesondere bevorzugte Emulgatoren sind Abil ® EM 90 (Cetyl Dimethicone Copolyol) oder Arlacel® 582 (Sorbitan Isostearate, PEG-2 Hydrogenated Castor Oil, Ozokerite, Hydrogenated Castor Oil, HLB = 5) oder Magnesium-, Aluminium-,Zink-stearat, insbesondere Mischungen hiervon. Ferner können die genannten Emulgatoren auch mit geeigneten Co-Emulgatoren kombiniert werden, so dass der gewünschte HLB- Wert vorliegt. Hierzu gehört z.B. Arlatone® T(V) , insbesondere in Kombination mit den bevorzugten W/O-Emulgatoren.

### B) O/W Emulgatoren

Als O/W Emulgatoren sind insbesondere solche mit einem HLB- Wert von 9- 18, bevorzugt 9-15 und insbesondere 9- 13 geeignet.

Hierzu gehören bevorzugt polyoxyethylierte Produkte wie (HLB- Werte rechts):

| | | |
|---|---|---|
| G - 2111 | Polyoxyethylen- oxypropylenoleat | 9,0 |
| G - 2125 | Tetrathylenglykolmonolaurat | 9,4 |
| Brij® 30 | Polyoxyethylenlaurylether | 9,5 |
| Tween® 61 | Polyoxyethylensorbitanmonostearat | 9,6 |
| Tween® 81 | Polyoxyethylensorbitanmonooleat | 10,0 |
| G - 3806 | Polyoxyethylencetylether | 10,3 |
| Tween® 65 | Polyoxyethylensorbitantristearat | 10,5 |
| Tween® 85 | Polyoxyethylensorbitantrioleat | 11,0 |
| G - 3910 | Polyoxyethylenoleyether | 12,2 |
| G - 2127 | Polyoxyethylenmonolaurat | 12,8 |
| Renex® 690 | Polyoxyethylenalkylarylether | 13,0 |
| | Polyethylenglykol-400-monolaurat | 13,1 |
| Cremophor® EL | Polyoxyethylen- Rizinusöl | 13,3 |
| G - 1284 | Polyoxyethylen- Rizinusöl | 13,3 |
| Tween® 21 | Polyoxyethylensorbitanmonolaurat | 13,3 |
| Renex® 20 | Polyoxyethylenester gemischter Fett' und Harzsäuren | 13,5 |
| G - 1441 | Polyoxyethylensorbit - Lanolinderivat | 14,0 |
| G - 7596J | Polyoxyethylensorbitanmonolaurat | 14,9 |
| Tween® 60 | Polyoxyethylensorbitanmonostearat | 14,9 |
| Tween® 80 | Polyoxyethylensorbitanmonooleat | 15,0 |
| Myrj® 49 | Polyoxyethylensorbitanmonostearat | 15,0 |
| G - 3720 | Polyoxyethylenstearylether | 15,3 |
| G - 3920 | Polyoxyethylenoleyether | 15,3 |
| Tween® 40 | Polyoxyethylensorbitanmonopalmitat | 15,6 |
| G - 2162 | Polyoxyethylen oxypropylenmonostearat | 15,7 |
| Cremophor® 0 | Polyoxyethylenfettalkoholether | 16,0 |
| G - 1471 | Polyoxyethylensorbit - Lanolinderivat | 16,0 |
| Myrj® 51 | Polyoxyethylenmonostearat | 16,0 |
| Cetomacrogol 1000 | Polyoxyethylenglykol-1 000-Monocetylether | 16,1 |
| Tween® 20 | Polyoxyethylensorbitanmonolaurat | 16,7 |
| Brji® 35 | Polyoxyethylenlaurylether | 16,9 |
| Myrj® 52 | Polyoxyethylenmonostearat | 16,9 |
| Myrj® 53 | Polyoxyethylenmonostearat | 17,9 |

Ferner sind bevorzugt auch:

| | | |
|---|---|---|
| Myrj® 45 | Polyoxyethylenglykolmonostearat | 11,2 |
| | Polyoxyethylenglykol-400-monooleat | 11,4 |
| Cremophor® AP- fest | Polyoxyethylenglykol- 400-monostearat | 11,6 |
| G - 2161 | Polyoxyethylenglykol400-monostearat | 11,6 |
| Brij® 721 P | Steareth-21 | 15,5 |
| Eumulgin®B1 | Ceteareth-12 | 13 |

Bevorzugt sind auch handelsübliche Gemische mit Co-Emulgatoren wie Fettalkoholen oder Glycerylestern wie Emulgator E 2149 (Steareth-7, Stearyl Alcohol, HLB = 11) oder Arlacel® 165 FL (Glyceryl Stearate, PEG-100 Stearate, HLB=11)
oder auch

| | | |
|---|---|---|
| Atlox® 3300 | Alkylarylsulfonattriethanolamioleat | 11,7 |

Weiterhin bevorzugt sind nichtionische und ionische Phosphate wie Cetyl- oder Stearylphosphate (HLB = 8, bzw. 10), Trilaureth-4 Phosphate (Hostaphat® KL 340, HLB ca. 13), Triceteareth-4 Phosphate (Hostaphat® KW 340, HLB = 10), (Poly)glycerylester wie Dermofeel®Q 182 S (Polyglyceryl-10-Distearat) oder PEG-4 Polyglyceryl-2 Stearate (Hostacerin® DGSB, HLB ca. 8), Polyglyceryl-2 PEG-10 Laurate (Hostacerin® DGL, HLB ca. 14), Polyglyceryl-3 Methyl Glucose Distearate (Tego Care® 450, HLB = 12) oder Gemische wie Eumulgin® VL 75 (Lauryl Glucoside, Polyglyceryl-2 Dipolyhydroxystearate, Glycerin, HLB ca. 13). Ferner bevorzugt sind Zuckerester wie Glucoseester z.B. Cetearyl Glucoside (Tego Care® CG 90, HLB = 11), Methyl Glucose Sesquistearate (Tego Care® PS, HLB = 12), PEG-20 Methyl Glucose Sesquistearate (Glucamate® SSE, HLB = 15) oder Mischungen mit Fettalkoholen wie Montanov® 82 (Cetearyl Alcohol, Coco Glucoside, HLB = 11), Montano® 14 (Myristyl Alkohol, Myristyl Glucoside, HLB = 10), Montanov® 202 (Arachidyl Alcohol, Behenyl Alcohol, Arachidyl Glucoside, HLB = 8) oder Sucroseester wie Sucrose Stearate (Crodesta® F 160, HLB = 14,5), Sucrose Cocoate (Crodesta® SL 40, HLB = 15) bzw. Mischungen hiervon, z.B. Crodesta® F 110 (Sucrose Stearate, Sucrose Distearate, HLB = 12).

Ferner können Sterol-Derivate als Emulgatoren eingesetzt werden, z.B. LanolinDerivate wie Laneth-20 (Polychol® 20, HLB = 14) oder Generol® RE 10 (PEG-10 Rapeseed Sterol, HLB = 12) oder Rizinusöl-Derivate wie PEG-40 Hydrogenated Castor Oil (Eumulgin® HRE 40, HLB = 15) oder PEG-36 Castor Oil (Arlatone® 650, HLB = 12,5), bzw. deren Gemische wie Arlatone® 980 (PEG-15 Hydroxystearate, PEG-25 Hydrogenated Castor Oil, HLB = 12,8).
Es können bevorzugt auch Siloxan-Derivate, insbesondere Polysiloxan-Polyether-Copolymere wie Abil® Care 85 (Dimethicone Copolyol, Caprylic/Capric Triglyceride, HLB = 10) eingesetzt werden.
Als ionische Emulgatoren können hier einwertige Salze z.B. von Fettsäuren oder Fettalkoholsulfaten eingesetzt werden z.B. Natriumstearat oder Triethanolaminostearat, Lanette® E (Sodium Cetearyl Sulfate) oder aber Phosphate wie Amphisol® K (Potassium Cetyl Phosphate) oder Glutamate, z.B. Hostapon® CCG (Sodium Cocoyl Glutamate) oder Lactylate, z.B. Crolactil® SSL (Sodium Stearoyl Lactylate).

Alle oben genannten Emulgatoren können auch in Gemischen mit Co-Emulgatoren, welche auch allgemein als Konsistenzgeber verwendet werden können, eingesetzt werden. Diese können aus der Gruppe der Fettalkohole, wie Stearyl Alcohol (Lanette® 18), Cetyl Alcohol (Lanette® 16), Myristyl Alcohol (Lanette® 14) oder Cetearyl Alcohol (Lanette® O) ausgewählt werden. Ferner geeignet sind Fettsäuren, z.B. Stearinsäure oder Glycerylester wie Glycerylstearat, insbesondere Glycerinmonostearat oder Glycerindistearat oder Gemische hiervon, z.B. Tegin® M.
Weiterhin können als Konsistenzgeber auch Wachse eingesetzt werden, z.B. Bienenwachs (Lunacera® alba), Kester®Wachs K82H (C₂₀₋₄₀- Alkylstearat) oder Lunacera® M (Micro Wax) oder Kohlenwasserstoffwachse wie Lunacera® P (Mineral Wax) sowie hydriertes Rizinusöl (Cutina® HR) oder synthetische Wachse wie Cetylpalmitat (Cutina® CP) oder Myristylmyristat (Crodamol® MM), oder Stearylstearate (Crodamol® SS).

Besonders bevorzugte O/W Emulgatoren sind:
Tego Care® 450( Polyglyceryl-3 Methyl Glucose Disterate, HLB = 12),
Eumulgin® B1 (Ceteareth-12, HLB =13) oder Mischungen hiervon.
Ferner bevorzugt sind auch die o.g. Hostaphat®-Produkte oder Abil® Care 85.

### IV.Zusatzstoffe

### 1) Zusatzwirkstoffe

Als Zusatzwirkstoffe können insbesondere Vitamine wie z.B. Tocopherolacetat (Vitamin E) oder Vitamin A, z.B. als Retinolpalmitat gewählt werden. Femer sind Elektrolyte wie Magnesiumsulfat oder Natriumchlorid ( Elektrolyte z.B. in Mengen von 0,2- 2% ) geeignet. Ferner können Polysaccharide, wie Glykosaminglycane, insbesondere Mucopolysaccharide eingesetzt werden. Hierzu gehören insbesondere nicht heparinoide Verbindungen wie z.B. Chondroitinsulfat oder Dermatansulfat oder Keratansulfat oder auch heparinoide Verbindungen wie Heparin, insbesondere deren Salze, z.B. Natriumsalze.
Ferner sind insbesondere Allantoin, D- Panthenol, Hyaluronsäure und/oder Zinkderivate wie Zincidone® (Zink PCA), Zinkglukonat oder Zinkoxid geeignet.
Die Mengen an einzelnen Zusatzwirkstoffen variieren und können z.B. von je jeweils 0,01- 20% bzw. 0,1- 6%, insbesondere 1- 5% bzw. 3- 5% betragen.
Darüber hinaus sind Vesikelbildner, insbesondere Lecithine und Analoge hiervon als Zusatzwirkstoffe geeignet. Hierzu gehören z.B. bekannte Stoffe (vgl. DE 42 05 548 C 2), insbesondere Phospholipide wie Lecithin (Ei- oder Soja-Lecithin), z.B. Phosal® 50 SA (ca. 50 % Soja-Lecithin), Phosphatidylcholin, -serin oder -diethanolamin sowie deren Mischungen.
Weitere geeignete Lecithin- analoge Komponenten sind Sphingolipide (z.B. Ceramide, Cerebroside, Sphingosin, Sphingomyelin), Phytosterole (im wesentlichen Gemische aus β-Sitosterol, Campesterol und Stigmasterol) sowie deren Derivate, insbesondere Ethoxylate wie Generol® 122 E 5 (PEG-5 Soybean Sterol), Gererol® R E5 (PEG-5 Rapeseed Sterol).
Ferner geeignet als Vesikelbildner sind polyethoxylierte Fettalkohole sowie Fettsäuren mit vorzugsweise 1-4 EO mit einem HLB-Wert von 2 bis 6 wobei der lipophile Rest bevorzugt aus C₁₆ bis C₁₈-Alkylketten besteht, Polyglycerolalkylether, Glucosyldialkylether, Saccharosediester, Kollagenhydrolysatester, quartäre Ammoniumverbindungen und Poloxamere. Besonders bevorzugt sind Ei- und/oder Soja-Lecithin (Phosal® 50SA), Phosphatidylcholin, Ceramide, Phytosterole und ethoxilierte Derivate hiervon mit einem Ethoxilierungsgrad von 5 bis 16 und polyoxyethylierte fettalkohole mit einem HLB-Wert von 2-6 oder Kombinationen hiervon, insbesondere von Phytosterolen der o.g. Art mit Ethoxylierungsprodukten hiervon.
Besonders bevorzugt hier ist Phosal® 50 SA.

Insbesondere bevorzugte Zusatzwirkstoffe sind:
a) Vitamin E (z.B. 0,1- 5, insbesondere 0,1-1%);
b) Magnesiumsulfat und /oder Natriumchlorid (z.B.0,1-5, insbesondere 0,1- 1%);
c) Mucopolysaccharide wie z.B. Chondroitinsulfat (z.B.0,1- 2, insbesondere 0,1-1%) sowie Heparin oder Heparinoide wie Heparin-Na ( z.B.0,1-2, insbesondere 0,1-1%);
d) Allantoin; Mengen wie vorstehend; z.B. 0,05-2%, bevorzugt 0,1-1%;
e) D- Panthenol; Mengen wie vorstehend; z.B. 0,1-10%, bevorzugt 1-5%;
f) Hyaluronsäure (z.B.0,001- 1, insbesondere 0,01- 0,1%);
g) Lecithine wie Phosal® 50 SA (z.B.0,5- 5%, insbesondere 1- 2%);
h)Zinkderivate wie Zinkgluconat oder Zink PCA (Zincidone®), z.B. 0,1-3, insbesondere 0,5-1%
i)ölabsorbierende Substanzen, z.B. Stärke-Derivate wie Natrasorb® HFB (Aluminium Starch, Octenylsuccinate, Acrylates Copolymer, Magnesium Carbonate) oder Acrylate, z.B. Micropearl® M 100 (Polymethyl Methacrylate), Micropearl® M 305 Methyl Methacrylate Copolymer) oder Sulfonate wie Biopol® OE (Sodium C8-16 Isoalkylsuccinyl Lactoglobulin Sulfonate).

Weiterhin können adstringierende und sebumregulierende Substanzen eingesetzt werden wie Acnacidol® 101 (Propylene Glykol, Hydroxydecanoic Acid), Asebiol® BT (Hydrolyzed Yeast Protein, Pyridoxine, Niacinamide, Glycerin, Panthenol, Propylene Glycol, Allantoin, Biotin), Lipacide® C8C0 (Caproyl Collagen Aminoacids), Sebosoft® (Glycerin, Aqua, PEG-8, Caprylyl Glycol, Sebacic Acid, Sodium Polyacrylate), Sepi Control® A5 (Capryloylglycine, Methylglycine, Cinnamonum zeylanicum).
Ferner können als Zusatzwirkstoffe weitere Pflanzenextrakte enthalten sein, z.B. Birkenblätterextrakt, Aloe-Vera-Extrakt, Ringelblumen-, Hibiskus-, Klettenwurzel-, Hamametis-, Wassernabelkraut-, Algen-, Quitten-, Wasserlilien-, Zimtextrakt.
Es können auch kühlende/beruhigende Wirkstoffe wie Frescolat® ML (Menthyl Lactate) oder Eashave® (Sodium Hyaluronate, Wheat Germ Extrakt, Saccharomyces Cerevisiae Extrakt) inkorporiert werden.
Darüber hinaus sind als Zusatzwirkstoffe durchblutungsfördernde Stoffe, z.B. Nikotinsäurederivate wie Methyl- oder Tocopherylnikotinat, Alpha- und Betahydroxysäuren und deren Derivate, z.B. Glykol-, Äpfel-, Zitronen-, Wein-Milchsäure, Salicylsäure, Isopropylbenzylsalicylate, C12 - 13 Alkyl Lactate (Cosmacol® ELI)oder auch antiphlogistische und antibakterielle Substanzen wie Triterpene, z.B. Ursolsäure, Glycyrrhizinsäure oder Glycyrrhetinsäure und deren Derivate, z.B. Stearyl Glycyrrhetinate, Kaliumglycyrrhinat; Pantothensäurederivate, z.B. D-Panthenol, Panthenyltriacetat; Allantoin; Bisabolol; Azulene, z.B. Cham-Azulene oder Guaj-Azulen; Phytosphingosine; Triclosan; Chlorhexidin-Derivate und/oder Antischuppenmittel, z. B. Climbazol oder Piroctone Olamine einsetzbar.
Daneben können in den erfindungsgemäßen Zusammensetzungen auch antioxidativ sowie zellschützend wirkende Stoffe wie Flavonoide, z.B. Rutin, Ferulasäure und deren Ester oder Isoflavone wie Soja-Isoflavone oder Coenzym Q 10 als wirksame Zusatzstoffe eingesetzt werden.
Ganz besonders bevorzugt werden Zusatzwirkstoffkombinationen aus a)- f) bzw. a)- i) oder a)+ c)- i). Diese können dann in geeigneter Weise mit den nachfolgend beschriebenen üblichen Zusatzstoffen kombiniert werden.

### 2) Übliche Zusatzstoffe

Diese sind vorzugsweise ausgewählt aus:
Antioxidantien, Parfümstoffen, Farbstoffen, UV-Filtern, Konservierungsmitteln und/oder Feuchthaltern, Stabilisatoren, Konsistenzgebern.
Die Antioxidantien können bevorzugt ausgewählt werden aus Butylhydroxytoluol, Butylhydroxyanisol, Ascorbylpalmitat, Tocopherol, evtl. in Kombination mit Synergisten wie in Controx® VP (Tocopherol,Lecithin, Ascorbyl Palmitate, Hydrogenated Palm Glycerides Citrate), Gallussäurealkylester wie Octyl-, Dodecyl- und Cetylgallat oder Kombinationen hiervon.
Parfümstoffe sind insbesondere ausgewählt aus etherischen Ölen. Ferner sind auch handelsübliche Parfüm-Kompositionen möglich wie z.B. Parfümöl Deliana . Etherische Öle, die für die erfindungsgemäßen Mittel geeignet sind, sind insbesondere die etherischen Öle ausgewählt aus Rosmarinöl, Orangenöl, Lavendelöl, Limettenöl, Zimtöl, Geraniumöl, Zedernholzöl, Rosenholzöl, Baldrianöl, Ylang-Ylang Öl, Citronellöl, Teebaumöl, Manukaöl, Eucalyptusöl, Minzöl, Lemongrasöl, Zypressenöl, Niaouliöl, Fichtennadelöl, Kiefemnadelöl, Campher, Menthol.
Diese Zusatzstoffe können auch als Zusatzwirkstoffe angesehen werden. Hierzu zählen dann auch essentielle ungesättigte Fettsäuren und deren Ester, z.B. Linol- oder Linolensäure, Glyceryl Linoleate, Glyceryl Linolenate eingesetzt werden.

Besonders bevorzugte Parfüm- bzw. Zusatz-Wirkstoffe sind ausgewählt aus etherischen Ölen, Pflanzenextrakten und -ölen, sebumregulierenden Stoffen, Feuchthaltemitteln, antiphlogistischen und antibakteriellen Substanzen, Vitaminen, ungesättigten essentiellen Fettsäuren oder Mischungen hiervon.
Bevorzugte Farbstoffe sind z.B.. Patentblau, Amidoblau, Orange RGL, Cochenillerot, Farbstoff FD+C Blue No.1 oder Titandioxid oder Chinolingelb.
Geeignete UV-Filter sind UVB, UVA und Breitbandfilter der folgenden Art:
UV-B Filter: Zimtsäureester, z.B. Octyl Methoxycinnamate (Eusolex® 2292, Neo Heliopan® AV, Parsol® MCX), Isoamyl p-Methoxycinnamate (Neo Heliopan® Galanga) sowie 4-Methylbenzyliden Camphor (Eusolex® 6300), Paraaminobenzoesäure und -ester wie N, N-Dimethyl-4-aminobenzoesäure-2-ethylhexylester (Eusolex® 6007, Octyl Dimethyl PABA), Homomenthylsacilylat (Homosalate, Eusolex® HMS), Octyl Salicylate (Neo Heliopan® OS), Octocrylene (Neo Heliopan® 303), Phenylbenzimidazolesulfonic Acid (Neo Heolipan® Hydro, Eusolex® 232)
UVA + UVB Filter für Breitbandabsorption wie Benzophenone-3 (Neo® Heliopan BB, Eusolex® 4360);
UV-A Filter wie Methyl Anthranilate (Neo Heliopan® MA), Butyl Methoxydibenzoylmethane (Parsol® 1789, Eusolex® 9020); Methylene Bis-Benzotriazolyl Tetramethylbutylphenol (Tinosorb® M), Bis-Ethylhexyloxyphenol Methoxyphenyltriazin (Tinosorb® S), Disodium Phenyl Dibenzimidazole Tetrasulfonate (Neo Heliopan® AP).

Insbesondere bevorzugt sind Octyl- oder Isoamyl p- Methoxycinnamate, Octocrylene, 4-Methylbenzyliden Camphor, Homosalate und/oder Butyl Methoxydibenzoylmethane und/oder Benzophenone-3.
Weiterhin geeignet sind anorganische UV-Filter wie Zinkoxid und Titandioxid insbesondere mikronisiert und/oder beschichtet, z.B. Z-Cote®, Tioveil®.
Als Konservierungsstoffe kommen lodopropynylbutylcarbamat, DMDM Hydantoin, Phenoxyethanol und weitere gebräuchliche Konservierungsstoffe, wie z.B. Sorbin- und Dehydracetsäure und deren Salze, Methyldibromoglutanonitril, etc. oder Kombinationen hiervon, oder andere Säuren wie Benzoe- oder Salicylsäure, oder Benzylalkohol oder Ester wie p-Hydroxy-benzoesäureester, z.B. Methyl-, Ethyl-, Propyl-, Butyl-, Iso-Butylparaben, bevorzugt Methyl-oder Propylparaben oder Mischungen hiervon oder Climbazol oder geeignete Kombinationen der genannten Stoffe wie z.B. Methyl-, Propylparaben und Sorbinsäure in Frage. Insbesondere geeignet sind Mischungen, z.B. aus DMDM Hydantoin und lodopropynylbutylcarbamat wie Glydant® plus.
Weiterhin geeignet sind Feuchthalter z.B. Polyalkohole wie Polyethylenglykol, Propylenglykol, Butylenglykol, Sorbitol, Glycerol oder Polymere, z.B. Polyquaternium-Typen wie Polyquaternium -39 (Merquat® plus 3330), Proteine wie Collagen oder dessen Hydrolysate, Aminosäuren, Harnstoff, D-Panthenol, pflanzliche Proteine wie z.B. aus Weizen, Soja oder Mandel oder deren Hydrolysate, z.B. Tritisol® (Hydrolyzed Wheat Protein), Polysaccharide wie z.B. Fucogel® 1000 (Biosaccharide Gum-1), Glucosaminoglykane, z.B. Hyaluronsäure oder sulfatierte Glucosaminoglykane wie Chondroitinsulfat, Dermatansulfat, Keratansulfat Heparansulfat, insbesondere deren Na-Salze oder Heparin-Na , Glukane, z.B. β-Glukan, z.B. Hafer β-Glukan (Drago-β-Glucan®), Mannane wie Konjac Mannane, Algenextrakte, z.B. Seamannin® SU oder handelsübliche Feuchhaltemittel wie z.B. Hydractin® (Glycerin, Aqua, Disodium Adenosine Triphosphate, Algin, Carica Papaya) oder Aquaderm® (Sodium PCA, Sodium Lactate, Fructose, Glycine, Niacinamid, Urea, Inositol), Salze, z.B. Natriumlactat, DL-2-Pyrrolidon-5-Carbonsäure, Na-Salz.
Bevorzugt sind Polyethylen-/ Propylenglykol oder Glycerin in Mengen von z.B.0,5- 10%, insbesondere 2- 5%, sowie Polysaccharid- Verbindungen wie Fucogel® 1000.
Als Stabilisatoren eignen sich zum einen Wachsprodukte wie z. B. Lunacera® alba (Cera alba), Lunacera® M (Cera Microcristallina), Cutina® HR (Hydrogenated Castor Oil) oder Silikonwachse, z.B. Abil® Wax 9800 (Stearyl Dimethicone). Ferner können auch Amerchol® CAB bestehend aus Vaseline und Lanolin, als Stabilisatoren eingesetzt werden.
Zum anderen sind als Stabilisatoren Komponenten zur pH- Wert Regulation verwendbar wie NaOH (z.B. 5% ig, z.B. in Mengen von 0,1- 4%, insbesondere 1-3%) oder Säuren wie z.B. Zitronensäure, Milchsäure oder Äpfelsäure oder EDTA- Na in geeigneter Menge als Komplexiermittel. Die Menge und Art der pH-Wert -Regulatoren hängt von den übrigen Zusatzstoffen ab und sind dem Fachmann geläufig.

Insbesondere ist als Stabilisator eine Kombination aus einem oder mehreren Acrylamiden, einem oder mehreren Acrylaten und einem oder mehreren Polysacchariden, insbesondere Stärke, bzw. Stärke-Derivaten geeignet, wobei jede Komponente in einer Menge von 0,05-8%, bevorzugt 0,1-5% enthalten sein kann.
Besonders bevorzugte Acrylamide sind Polyacrylamid, z.B. Flocare® T 920 GC oder Polyacrylamidhaltige Gemische wie Sepigel® 305 (Polyacrylamide, C13-14 Isoparaffin, Laureth-7), Sepigel® 501 (Acrylamides Copolymer, Mineral Oil, C13-14 Isoparaffin, Polysorbate 85), Sepigel® 502 (C13-14 Isoparaffin, Isostearyl Isostearate, Sodium Polyacrylate, Polyacrylamide, Polysorbat 20), Creagel® EZ DC (Polyacrylamide, Polydecene, Dimethicone Copolyol), Creagel® EZ 5 (Polyacrylamide, Polydecene, Laureth-5).
Als Acrylate werden Carboxy-Vinyl-Mischpolymerisate mit hohem Molekulargewicht (1 - 3 Mio.) sowie deren Copolymere eingesetzt, insbesondere nach Neutralisierung durch Alkali. Bevorzugt sind die unter dem INCI-Namen Carbomer bekannten Carbopol®-Typen, z.B. Carbopol® 910, 934, 940, 941, 954, 980, 981, 2984, 5984 oder Carbopol® ETD 2001, 2050 oder Synthalen® K, L, M oder die bereits neutralisierten Carbomere wie PNC® 400, 410, 430 (INCI: Sodium Carbomer). Es können auch Acrylat-Copolymere eingesetzt werden, z.B. Arylates/C10-30 Alkyl Acrylate Crosspolymer, bekannt als Carbopol® 1342, 1382, ETD 2020, Pemulen® TR-1, TR-2.
Als Stärke, bzw. Stärke-Derivate sind insbesondere folgende Stoffe geeignet: Reis-, Weizen-, Mais- und Kartoffelstärke.
Besonders bevorzugt sind hydrophobisch modifizierte Stärken wie Aluminium Starch Octenylsuccinate (Dry Flo® PC, Fluidamid® DF 12) oder dessen Gemische wie Natrasorb® HFB (Aluminium Starch Octenylsuccinate, Acrylates Copolymer, Magnesium Carbonate), ASO/MM3® (Aluminium Starch Octenylsuccinate, Magnesium Myristate), Dry Flo® Elite LL (Aluminium Starch Octenylsuccinate, Lauroyl Lysine), Facemat® (Aluminium Starch Octenylsuccinate, Mica, Zea Mays (Com) Starch, Silica, Titanium Dioxide, Zink Oxide).
Ganz besonders bevorzugt sind Dry Flo® PC und Natrasorb® HFB.

Die oben genannte Kombination aus Stabilisatoren kann auch insbesondere bei Einsatz von O/W- Emulgatoren verwendet werden.
Besonders bevorzugt ist hier Sepigel®305 oder 501, PNC®410/400 oder Carbopol®ETD2020 und Dry Flo®PC oder Natrasorb®HFB, insbesondere Kombinationen hiervon.

Bevorzugt werden Konservierungsstoffe, Antioxidantien, Farb- und Parfümstoffe zusammen mit Stabilisatoren und Feuchthaltern und Zusatzwirkstoffen ggf. in Verbindung mit pH- Regulatoren als Zusatzstoffe, insbesondere bevorzugt in Verbindung mit den o.g. Zusatzwirkstoffen eingesetzt.
Die Mengen an Zusatz- bzw. Wirkstoffen können in den angegebenen Bereichen variieren.
Bevorzugt sind Zusatzstoffe ausgewählt aus Zusatzwirkstoffen wie Vitaminen, Elektrolyten wie Magnesiumsulfat und Natriumchlorid, Allantoin, D- Panthenol, Hyaluronsäure, Mucopolysacchariden wie z.B. Heparinoiden und Nicht-Heparinoiden sowie deren Mischungen, sowie Parfümstoffen, Konservierungsmittel und Feuchthaltemittel.
Diese können bevorzugt mit Wachsen und/oder Lecithinen wie insbesondere Phosal®50SA kombiniert werden.

Das beschriebene Zwiebelextrakt- enthaltende Mittel kann hergestellt werden, indem man Zwiebelextrakt , bevorzugt mit dort löslichen Zusatzstoffen vereinigt, sodann die Fettphase herstellt, wobei bevorzugt dort lösliche Zusatzstoffe inkorporiert werden können, und dann die Wasserphase bereitet, welche bevorzugt dort lösliche Zusatzstoffe und insbesondere Alkohole der oben genannten Art und Menge aufweist, und sodann die Wasser- und die Fettphase bei Temperaturen von 60 bis 90°C zusammen mit einem oder mehreren Emulgatoren oder Gemischen hiervon oder mit Co- Emulgatoren emulgiert, ggf. homogenisiert und nach Abkühlen (z.B. bei 20-50°C ) die Zusatzstoffe, sofern vorhanden, hinzufügt und in geeigneter Weise aufarbeitet, z.B. durch Homogenisieren.
Andererseits kann der Zwiebelextrakt zusammen mit den Zusatzstoffen in die Fett(öl)phase inkorporiert werden, wobei ggf. auch Lösungsmittel ,insbesondere Alkohole der oben genannten Art und Menge hinzugefügt werden können.
Als Zwiebelextrakt wird der jeweils gewünschte wässrige, alkoholische, alkoholisch-lösungsmittelhaltige oder wässrig-alkoholische oder Mischungen hiervon, z.B. im Verhältnis 3:1 bis 1:3 z.B. der beiden letztgenannten , in den angegebenen Mengen gewählt.
Ein wie oben beschriebenes hergestelltes Mittel eignet sich insbesondere z.B. als dermatologische Zusammensetzung zur Pflege, Behandlung oder Vorbeugung von geschädigtem Hautgewebe, insbesondere Narbengewebe, oder erschlafftem Gewebe wie z.B. Schwangerschaftsstreifen, oder auch von geschädigtem Hautgewebe, welches entstanden sein kann aufgrund von Schnittwunden, Operationswunden, Verbrennungen oder durch altersbedingte Degeneration.
Das geschädigte Hautgewebe zeigt schon nach kurzer Zeit eine Verbesserung der vernarbten oder geschädigten Anteile und kann durch die neue Ölphasen enthaltende Formulierung über hinaus noch wesentlich weicher, geschmeidiger und elastischer erhalten werden, als ohne diesen pflegenden, hautregenerierenden Zusatz.
Somit kann das Mittel auch bei Narben nach Schönheitsoperationen oder auch zur Behandlung von Aknenarben überraschenderweise mit überaus regenerativem Erfolg eingesetzt werden.

Die Erfindung wird anhand der nachfolgenden Beispiele näher erläutert.
In den Beispielen 1-6 wurde ein wässrig- ethanolischer Zwiebelextrakt, insbesondere mit einem Anteil von 13-20% Ethanol und in den Beispielen 7,8 ein Alkohol-haltiger, nämlich Ethanol und mittelkettige Trigycerid (ca. 60%) enthaltender Zwiebelextrakt eingesetzt. In den Beispielen 9, 10 ist die Wirksamkeit anhand medizinischer Anwendungsbeobachtungen aufgezeigt.

### Beispiel 1

| | |
|---|---|
| Aqua purificata | 34,7879 |
| Magnesiumsulfat | 0,7000 |
| Arlacel 582 Nena | 8,0000 |
| Arlatone TV | 1,5000 |
| Lunacera alba | 2,0000 |
| Lunacera M | 2,0000 |
| Magnesiumsterat | 2,0000 |
| Amerchol CAB | 5,0000 |
| Cetiol OE | 5,0000 |
| Paraffinum Perliquidum | 10,0000 |
| Cosmacol ELI | 3,0000 |
| Nachtkerzenöl | 2,0000 |
| Tocopherolacetat | 0,5500 |
| Oxynex 2004 | 0,0500 |
| Glydant plus | 0,2000 |
| Propylenglykol | 4,0000 |
| D- Panthenol | 5,5000 |
| Allantoin | 1,0000 |
| Hyaluronsäure | 0,0100 |
| Chondroitinsulfat | 0,2000 |
| Zwiebelextrakt | 10,0000 |
| Farbstoff Pigmentpaste | 2,0000 |
| Farbstoff FD+C Blue No. 1 | 0,0003 |
| Parfümöl Deliana | 0,5000 |

### Beispiel 2

| | |
|---|---|
| Aqua purificata | 51,7879 |
| Natriumchlorid | 0,5000 |
| Abil EM 90 | 2,5000 |
| Abil Wax 9800 | 1,0000 |
| Jojobaöl | 1,0000 |
| Arlamol HD | 5,0000 |
| Cetio OE | 4,0000 |
| Lunacera M | 0,6000 |
| Cutina HR Powder | 0,4000 |
| Cosmacol ELI | 3,0000 |
| Nachtkerzenöl | 2,0000 |
| Tocopherolacetat | 0,5500 |
| Oxynex 2004 | 0,0500 |
| Dow Corning 345 Fluid | 4,0000 |
| Glydant plus | 0,2000 |
| Propylenglykol | 4,0000 |
| D- Panthenol | 5,5000 |
| Allantoin | 1,0000 |
| Phosal SA 50 Neu | 2,0000 |
| Hyaluronsäure | 0,0100 |
| Chondroitinsulfat | 0,4000 |
| Zwiebelextrakt | 10,0000 |
| Farbstoff FD+C Blue No. 1 | 0,0002 |
| Parfümöl Deliana | 0,5000 |

### Beispiel 3

| | |
|---|---|
| Aqua purificata | 38,7900 |
| Carbopol ETD 2020 | 0,2500 |
| Tego Care 450 | 3,0000 |
| Eumulgin B1 | 2,0000 |
| Lunacera alba | 2,0000 |
| Kester Wachs K82H | 3,0000 |
| Arlamol HD | 1,5000 |
| Cetiol SB 45 | 2,0000 |
| Finsolv TN | 3,0000 |
| Dow Coming Fluid 345 | 2,0000 |
| Cosmacol ELI | 3,0000 |
| Nachtkerzenöl | 2,0000 |
| Tocopherolacetat | 0,5500 |
| Oxynex 2004 | 0,0500 |
| Propylenglykol | 4,0000 |
| Glydant plus | 0,2000 |
| NaOH 5%ig | 2,8000 |
| Trilon BD | 0,1000 |
| Panthenol | 5,5000 |
| Hyaluronsäure Na- Salz | 0,0100 |
| Phosal SA 50 | 2,0000 |
| Allantoin | 1,0000 |
| Zincidone | 0,5000 |
| Fucogel 1000 | 5,0000 |
| Natrasorb HFB | 3,0000 |
| Zwiebelfluidextrakt | 10,0000 |
| Farbstoff Pigment Paste weiß | 2,0000 |
| Sepigel 305 | 0,5000 |
| Parfümöl Deliana | 0,5000 |

### Beispiel 4

| | |
|---|---|
| Aqua purificata | 33,5900 |
| Tego Care 450 | 3,0000 |
| Eumulgin B1 | 2,0000 |
| Lunacera alba | 1,0000 |
| Kester Wachs K82H | 3,0000 |
| Arlamol HD | 2,5000 |
| Cetiol SB 45 | 2,0000 |
| Finsolv TN | 3,0000 |
| Dow Corning Fluid 345 | 2,0000 |
| Cosmacol ELI | 3,0000 |
| Nachtkerzenöl | 2,0000 |
| Tocopherolacetat | 0,5500 |
| Oxynex 2004 | 0,0500 |
| PNC410 | 0,2000 |
| Propylenglykol | 4,0000 |
| Glydant plus | 0,2000 |
| NaOH 5%ig | 0,9000 |
| Panthenol | 5,5000 |
| Hyaluronsäure Na- Salz | 0,0100 |
| Phosal SA 50 | 2,0000 |
| Allantoin | 1,0000 |
| Zincidone | 0,5000 |
| Fucogel 1000 | 5,0000 |
| Sepigel305 | 0,5000 |
| Zwiebelfluidextrakt | 10,0000 |
| Farbstoff Pigment Paste weiß | 2,0000 |
| Dry Flow PC | 5,0000 |
| Parfümöl Deliana | 0,5000 |

### Beispiel 5

| | |
|---|---|
| Aqua purificata | 41,0900 |
| Carbopol ETD2020 | 0,2000 |
| Dermofeel Q182S | 3,0000 |
| Eumulgin B1 | 2,0000 |
| Lunacera alba | 2,0000 |
| Kester Wachs K82H | 3,0000 |
| Arlamol HD | 5,0000 |
| Cetiol SB 45 | 2,0000 |
| Finsolv TN | 3,0000 |
| Dow Corning Fluid 345 | 2,0000 |
| Cosmacol ELI | 3,0000 |
| Tocopherolacetat | 0,5500 |
| Oxynex 2004 | 0,0500 |
| Propylenglykol | 4,0000 |
| Glydant plus | 0,2000 |
| NaOH 5%ig | 1,6000 |
| Panthenol | 5,5000 |
| Hyaluronsäure Na- Salz | 0,0100 |
| Phosal SA 50 | 2,0000 |
| Allantoin | 1,0000 |
| Zincidone | 0,5000 |
| Fucogel 1000 | 5,0000 |
| Sepigel305 | 1,0000 |
| Zwiebelfluidextrakt | 5,0000 |
| Farbstoff Pigment Paste weiß | 1,0000 |
| Parfümöl Deliana | 0,5000 |
| Natrasorb HFB | 3,0000 |

### Beispiel 6

| | |
|---|---|
| Aqua purificata | 46,1900 |
| Carbopol ETD2020 | 0,2000 |
| Dermofeel Q182S | 3,0000 |
| Eumulgin B1 | 2,0000 |
| Lunacera alba | 2,0000 |
| Kester Wachs K82H | 1,0000 |
| Arlamol HD | 5,0000 |
| Cetiol SB 45 | 2,0000 |
| Finsolv TN | 3,0000 |
| Dow Corning Fluid 345 | 2,0000 |
| Cosmacol ELI | 3,0000 |
| Tocopherolacetat | 0,5500 |
| Oxynex 2004 | 0,0500 |
| PNC 410 | 0,6000 |
| Propylenglykol | 4,0000 |
| Glydant plus | 0,2000 |
| NaOH 5%ig | 1,6000 |
| Panthenol | 5,5000 |
| Hyaluronsäure Na- Salz | 0,0100 |
| Phosal SA 50 | 2,0000 |
| Allantoin | 1,0000 |
| Zincidone | 0,5000 |
| Fucogel 1000 | 5,0000 |
| Sepigel305 | 0,3000 |
| Zwiebelfluidextrakt | 5,0000 |
| Farbstoff Pigment Paste weiß | 1,0000 |
| Parfümöl Deliana | 0,5000 |
| Natrasorb HFB | 3,0000 |

### Beispiel 7

| | |
|---|---|
| Miglyol 812 | 73,9000 |
| Zwiebelextrakt | 2,0000 |
| Tocopherolacetat | 1,1000 |
| Nachtkerzenöl | 2,0000 |
| Jojobaöl | 5,0000 |
| Cosmacol ELI | 5,0000 |
| Cetiol PGL | 10,0000 |
| Parfümöl Deliana | 1,0000 |

### Beispiel 8

| | |
|---|---|
| Miglyol 812 | 86,9000 |
| Zwiebelextrakt | 2,0000 |
| Tocopherolacetat | 1,1000 |
| Nachtkerzenöl | 2,0000 |
| Jojobaöl | 3,0000 |
| Cosmacol ELI | 5,0000 |
| Parfümöl Deliana | 0,7000 |

### Beispiel 9 Medizinische Anwendungsbeobachtungen mit Patienten mit unterschiedlichsten Narben

Bei 338 Patienten (62 % weiblich) im durchschnittlichen Alter von 41 Jahren waren Schnittwunden (45), Schürfwunden (14), Operationswunden (240), Verbrennungsnarben (18), Aknenarben (6) oder andere Narben (15) im Kopf-/Halsbereich (65), Armen (135), Beinen (84), am Oberkörper/Brust (27), Oberkörper/Rücken (14) oder anderen Stellen (54) über einen Zeitraum von < 1 Monat (106) bis 3 Monate (117) bzw. > 3 Monate vorhanden. Diese Patienten wurden über einen Zeitraum bis 6 Monate mit einem Produkt gemäß vorliegendem Beispiel 1 durch Auftragen auf die betroffene Hautstelle im Durchschnitt 2x täglich behandelt. Vor der Behandlung war in etwa 15 % der Fälle eine Anwendung bekannter Produkte wie Zwiebelextrakt-Gel (Contractubex) oder Ketofibrase ohne weiteren Erfolg vorgenommen worden. Während der erfindungsgemäßen Behandlung erfolgte eine Beurteilung hinsichtlich der Hautglättung (Aussehen der Narbe wie übrige Haut bzw. leicht oder stark verhärtet, Reduktion Narbentiefe, -länge), der Hautverträglichkeit (Verteilbarkeit, Einziehvermögen, Geruch, Hautgefühl). Die Ergebnisse sind in nachfolgender Tabelle 1 dargestellt und zeigen, dass das erfindungsgemäße Produkt bei über 90% der Fälle zu einem positiven Ergebnis geführt hatte.

**Tabelle 1**

| Narbenglättung: | Beurteilung Arzt | Beurteilung Patient |
|---|---|---|
| a) Sehr gut | 45,0 % | 46,7 % |
| b) gut | 47,6 % | 44,7 % |
| c) mäßig | 5,6 % | 8,0 % |
| *Gesamt-Urteil a)* + *b)* | *91,4 %* | *92,6 %* |

| Hautverträglichkeit: | | |
|---|---|---|
| a) sehr gut | 75,9 % | 70,3 % |
| b) gut | 23,2 % | 28,5 % |
| c) mäßig | 0,9 % | 1,2 % |
| *Gesamt-Urteil a)* + *b)* | *99,1 %* | *98,8 %* |

### Beispiel 10: Medizinische Anwendungsbeobachtung bei der Vorbeugung und Behandlung von Schwangerschaftsstreifen

741 Probanden wurden ab der 16. Schwangerschaftswoche im Hinblick auf die Ausbildung, Verhinderung von Schwangerschaftssteifen über einen Zeitraum von 5-7 Monaten bis 8 Wochen nach der Geburt beobachtet und mit einem Produkt gemäß Beispiel 2 oben auf Bauch, Brust und Oberschenkel 1-2x täglich behandelt. Zu beginn der Studie lagen bei 62,5 % keine Streifen, bei 37, 3 % Streifen ( davon 19,2 % leicht, 14,7 % viele leichte, 3,4 % viele starke) vor. Von den Probandinnen waren 65,8 % Erstgebärende (davon 7 % mit Steifen) und 34,1 % Mehrfachgebärende (davon 29,1 % mit Streifen). Nach dem Beobachtungszeitraum ergab sich, dass trotz einer durchschnittlichen Gewichtszunahme von ca. 12 kg bei 35.9 % keine Schwangerschaftsstreifen sich entwickelten und nur bei 37,1 % wenige leichte Streifen auftraten, während bei 21,3 % viele leichte und nur bei 5,7 % starke Streifen festgestellt wurden. Das erfindungsgemäße Produkt ist daher außerordentlich gut geeignet zur wirksamen Verhinderung bzw. Reduktion von Schwangerschaftsstreifen, was sich aus den in Tabelle 2 genannten Beurteilungswerten ( Kriterien analog Beispiel 9 ) eindeutig ergibt.

**Tabelle 2**

| Entwicklung Streifen: | Beurteilung Arzt | Beurteilung Patient |
|---|---|---|
| a) Sehr gut | 53,0 % | 51,1 % |
| b) gut | 39,0 % | 40,1 % |
| c) mäßig | 7,0 % | 6,0 % |
| d) schlecht | 1,0 % | 2,3 % |
| *Gesamt-Urteil a)* + *b)* | *92,0 %* | *91,1 %* |

| Hautverträglichkelt: | Beurtellung Arzt | Beurteilung Patient |
|---|---|---|
| a) sehr gut | 69,6 % | 75,7 % |
| b) gut | 28,1 % | 22,9 % |
| c) mäßig | 0,9% | 0,9 % |
| *Gesamt-Urteil a)* + *b)* | *97,7 %* | *98,6 %* |

## Patentansprüche

1. Einen Zwiebelextrakt enthaltendes Mittel zur topischen Anwendung, **dadurch gekennzeichnet, dass** es eine Fett(öl)-phase, Zusatzstoffe ausgewählt aus Konsistenzgebem, Farbstoffen, Parfümstoffen, Feuchthaltem, Antioxidantien, Konservierungsmitteln, Stabilisatoren, Zusatzwirkstoffen, sowie einen Zwiebelextrakt enthält..

2. Mittel gemäß Anspruch 1, **dadurch gekennzeichnet, dass** es 70-99% Fett(öl)-phase, 0,1-20% Zusatzstoffe und 1-20% Zwiebelextrakt aufweist.

3. Mittel gemäß Anspruch 1, **dadurch gekennzeichnet, dass** es eine Fett(öl)phase, Zusatz-stoffe, ausgewählt aus Konsistenzgebem, Farbstoffen, Parfümstoffen, Feuchthaltern, Antioxidan-tien, Konservierungsmitteln, Stabilisatoren, Zusatzwirkstoffen und einen Zwiebelextrakt enthält sowie weiterhin eine wässrige Phase und einen oder mehrere W/O- oder O/W-Emulgatoren oder Mischungen oder Mischungen mit Co- Emulgatoren aufweist..

4. Mittel gemäß einem der Ansprüche 1-3, **dadurch gekennzeichnet, dass** der Zwiebelextrakt ein Wasser, Alkohol oder Wasser-Alkohol enthaltender Zwiebelextrakt ist.

5. Mittel gemäß Anspruch 3 oder 4, **dadurch gekennzeichnet, dass** es 5- 70% Fett(öl)phase, 0,1- 15% W/O- oder O/W- Emulgatoren oder Mischungen oder Mischungen mit Co-Emulgatoren, 1- 20% Zwiebelextrakt, 0,1- 35% Zusatzstoffe und als Rest Wasser oder ein Wasser/Alkohol-Gemisch enthält.

6. Mittel gemäß einem der Ansprüche 3-5 , **dadurch gekennzeichnet, dass** es eine Fett(ö)phase in einer Menge von 5- 70%, einen oder mehrere W/O-Emulgatoren in einer Gesamtmenge von 0,5- 15%, sowie deren Mischungen oder Mischungen mit Co- Emulgatoren, sowie 0,5- 25% Zusatzstoffe,1-15% Zwiebelextrakt und als Rest Wasser oder ein Wasser/Alkohol-Gemisch aufweist.

7. Mittel gemäß Anspruch 6, **dadurch gekennzeichnet, dass** es 5- 55% Ölphase, 0,5- 10% eines oder mehrerer W/O- Emulgatoren oder deren Mischungen oder Mischungen mit Co- Emulgatoren, 0,1- 25% Zusatzstoffe ,1- 10% Zwiebelextrakt und als Rest Wasser oder ein Wasser/Alkohol-Gemisch aufweist.

8. Mittel gemäß einem der Ansprüche 3-5, **dadurch gekennzeichnet, dass** es 5-40% Fett(öl)-phase, 0,1- 15% eines oder mehrerer O/W- Emulgtoren oder Mischungen hiervon mit Co-Emulgatoren , 0,1- 32% Zusatzstoffe, 1- 10% Zwiebelextrakt und als Rest Wasser aufweist.

9. Mittel gemäß einem der Ansprüche 1-8, **dadurch gekennzeichnet, dass** es als Zwiebel-extrakt einen wässrig- ethanolischen Extrakt enthält.

10. Mittel gemäß Anspruch 9, **dadurch gekennzeichnet, dass** der Zwiebelextakt Wasser und 10-40% Ethanol enthält.

11. Mittel gemäß einem der Ansprüche 1-8, **dadurch gekennzeichnet, dass** als Zwiebelextrakt ein alkoholischer Extrakt enthalten ist, welcher 10-80% eines Lösungsmittels, ausgewählt aus Fettsäureestern, Triglyceriden, Kohlenwasserstoffen oder Mischungen hiervon aufweist.

12. Mittel gemäß einem der Ansprüche 1-8, **dadurch gekennzeichnet, dass** der Zwiebel-extrakt eine Mischung aus einem Wasser/Ethanol-Zwiebelextrakt mit 10-40% Ethanol und einem alkoholischen Zwiebelextrakt mit 10-80% eines Lösungsmittels, ausgewählt aus Fettsäure-estern, Triglyceriden, Kohlenwasserstoffen oder Mischungen hiervon ist.

13. Mittel gemäß einem der Ansprüche 1-12, **dadurch gekennzeichnet, dass** die Öl(Fett)phase ausgewählt ist aus Kohlenwasserstoffen, Fettalkoholen, Fettalkoholethern oder -estern, (Polyol)fettsäureestern, Triglyceriden, natürlichen Ölen , natürlichen Fetten, Wachsen, Silikonölen, Silikonwachsen oder Mischungen hiervon.

14. Mittel gemäß einem der Ansprüche 1- 13, **dadurch gekennzeichnet, dass** es als ölphase flüssige Paraffine; Milchsäureester, Fettalkoholether, Nachtkerzenöl, Silikonöl oder Mischungen hiervon aufweist.

15. Mittel gemäß einem der Ansprüche 3-14, **dadurch gekennzeichnet, dass** der W/O-Emulgator einen HLB- Wert von 1-8 und der O/W- Emulgator einen HLB- Wert von 9-18 aufweist oder ein ionischer O/W- Emulgator ist.

16. Mittel gemäß einem der Ansprüche 3-7 oder 9-15, **dadurch gekennzeichnet, dass** es als Emulgator Sorbitan-Derivate, polyethoxylierte, Fettsäuren/Alkohole/Ester/ Triglyce-ride, (Poly)glycerylderivate, Polyolester, Glucose-Derivate, Pentaerythrit- Derivate, Alkyl-phenole, (Block)Polymere, Siloxane, Fettsäuresalze oder Mischungen hiervon enthält.

17. Mittel gemäß einem der Ansprüche 3-7 oder 9-16, **dadurch gekennzeichnet, dass** es als Emulgator Abil® EM 90, Arlacel ®582 und Magnesiumstearat oder Mischungen hiervon aufweist.

18. Mittel gemäß einem der Ansprüche 3-5 oder 8-15, **dadurch gekennzeichnet, dass** der Emulgator ausgewählt ist aus polyoxyethylierten Produkten, nichtionischen und ionischen Phosphaten, ionischen einwertigen Salzen, (Poly)glycerylestern, Zuckerestern, Sterolderi-ivaten, Rizinusöl-Derivaten, Siloxanen oder Gemischen hiervon oder Mischungen mit Co- Emuigatoren hiervon.

19. Mittel gemäß Anspruch 18, **dadurch gekennzeichnet, dass** es als Emulgator Tego Care® 450, Eumulgin® B1 oder Mischungen hiervon enthält.

20. Mittel gemäß einem der Ansprüche 3-5, 8-16 oder 18,19, **dadurch gekennzeichnet, dass** als Stabilisator eine Mischung aus Acrylamiden, Acrylaten und Polysacchariden verwendet wird.

21. Mittel gemäß einem der Ansprüche 1- 20, **dadurch gekennzeichnet, dass** es als Zusatzstoffe, solche ausgewählt aus Vitaminen, Elektrolyten, Allantoin, D-Panthenol, Hyaluronsäure, Mucopolysacchariden, Farbstoffen, Parfümstoffen, Konservierungs-mittel, Feuchthalter aufweist.

22. Mittel gemäß einem der Ansprüche 1- 21, **dadurch gekennzeichnet, dass** es zusätzlich Wachsprodukte aufweist.

23. Mittel gemäß einem der Ansprüche 1- 22, **dadurch gekennzeichnet, dass** es als Zusatzstoffe weiterhin Lecithine enthält.

24. Mittel gemäß einem der Ansprüche 1-23 zur Pflege von geschädigtem Hautgewebe.

25. Verfahren zur Herstellung von Mitteln gemäß einem der Ansprüche 1- 23, **dadurch gekennzeichnet, dass** man den Zwiebelextrakt, worin darin lösliche Zusatzstoffe ein-gearbeitet sein können, und die Fettphase herstellt, wobei dort lösliche Zusatzstoffe inkorporiert werden können, und dann die Wasserphase bereitet, welche dort lösliche Zusatzstoffe und insbesondere Alkohole aufweisen kann, und sodann die Wasser- und die Fettphase bei Temperaturen 60 bis 90°C zusammen mit einem oder mehreren Emulgatoren oder Gemischen hiervon oder mit Co- Emulgatoren emulgiert und nach Abkühlen die Zusatzstoffe, sofern vorhanden, hinzufügt und in geeigneter Weise aufarbeitet oder den Zwiebelextrakt zusammen mit den Zusatzstoffen in die Fett(öl)-phase inkorporiert , wobei auch Alkohole als Lösungsmittel hinzugefügt werden können.

26. Verwendung eines Mittels gemäß einem der Ansprüche 1- 23 zur Herstellung von Zusammensetzungen zur Behandlung, Pflege oder Vorbeugung von geschädigtem Hautgewebe.

27. Verwendung gemäß Anspruch 26, **dadurch gekennzeichnet, dass** das geschädigte Hautgewebe Narbengewebe ist.

28. Verwendung gemäß Anspruch 27, **dadurch gekennzeichnet, dass** das Narbengewebe solches infolge von Akne oder Schönheitsoperationen entstandenes Gewebe ist.

29. Verwendung gemäß Anspruch 26, **dadurch gekennzeichnet, dass** das geschädigte Hautgewebe Schwangerschaftsstreifen sind.

30. Verwendung gemäß Anspruch 26, **dadurch gekennzeichnet, dass** das geschädigte Hautgewebe solches entstanden aufgrund von Schnittwunden, Operationswunden, Verbrennungen oder durch altersbedingte Degeneration entstandenes geschädigtes Hautgewebe ist.

## Claims

1. Agent for topical application containing an onion extract, **characterised in that** it comprises a fat (oil) phase, additives chosen from consistency-imparting agents, dyes, perfume substances, humectants, antioxidants, preservatives, stabilisers, active ingredients, and an onion extract.

2. Agent according to claim 1, **characterised in that** it has 70-99% fat (oil) phase, 0.1-20% additives and 1-20% onion extract.

3. Agent according to claim 1, **characterised in that** it comprises a fat (oil) phase, additives chosen from consistency-imparting agents, dyes, perfume substances, humectants, antioxidants, preservatives, stabilisers, active ingredients and an onion extract, and also an aqueous phase and one or more W/O or O/W emulsifiers or mixtures or mixtures with co-emulsifiers.

4. Agent according to one of claims 1-3, **characterised in that** the onion extract is an onion extract containing water, alcohol or water-alcohol.

5. Agent according to claim 3 or 4, **characterised in that** it comprises 5-70% fat (oil) phase, 0.1-15% W/O or O/W emulsifiers or mixtures or mixtures with co-emulsifiers, 1-20% onion extract, 0.1-35% additives, and water or a water/alcohol mixture as the remainder.

6. Agent according to one of claims 3-5, **characterised in that** it has a fat (oil) phase in an amount of 5-70%, one or more W/O emulsifiers in a total amount of 0.5-15%, and mixtures thereof or mixtures with co-emulsifiers, and 0.5-25% additives, 1-15% onion extract and water or a water/alcohol mixture as the remainder.

7. Agent according to claim 6, **characterised in that** it has 5-55% oil phase, 0.5-10% of one or more W/O emulsifiers or mixtures thereof or mixtures with co-emulsifiers, 0.1-25% additives, 1-10% onion extract and water or a water/alcohol mixture as the remainder.

8. Agent according to one of claims 3-5, **characterised in that** it has 5-40% fat (oil) phase, 0.1-15% of one or more O/W emulsifiers or mixtures thereof with co-emulsifiers, 0.1-32% additives, 1-10% onion extract and water as the remainder.

9. Agent according to one of claims 1-8, **characterised in that** it comprises an aqueous-ethanolic extract as onion extract.

10. Agent according to claim 9, **characterised in that** the onion extract comprises water and 10-40% ethanol.

11. Agent according to one of claims 1-8, **characterised in that** an alcoholic extract which has 10-80% of a solvent chosen from fatty acid esters, triglycerides, hydrocarbons or mixtures thereof is present as onion extract.

12. Agent according to one of claims 1-8, **characterised in that** the onion extract is a mixture of a water/ethanol onion extract with 10-40% ethanol and an alcoholic onion extract with 10-80% of a solvent chosen from fatty acid esters, triglycerides, hydrocarbons or mixtures thereof.

13. Agent according to one of claims 1-12, **characterised in that** the oil(fat) phase is chosen from hydrocarbons, fatty alcohols, fatty alcohol ethers or esters, (polyol) fatty acid esters, triglycerides, natural oils, natural fats, waxes, silicone oils, silicone waxes or mixtures thereof.

14. Agent according to one of claims 1-13, **characterised in that** it has liquid paraffins, lactic esters, fatty alcohol ethers, evening primrose oil, silicone oil or mixtures thereof as the oil phase.

15. Agent according to one of claims 3-14, **characterised in that** the W/O emulsifier has a HLB value of 1-8 and the O/W emulsifier has a HLB value of 9-18, or is an ionic O/W emulsifier.

16. Agent according to one of claims 3-7 or 9-15, **characterised in that** it comprises sorbitan derivatives, polyethoxylated fatty acids/alcohols/esters/triglycerides, (poly)glyceryl derivatives, polyolesters, glucose derivatives, pentaerythritol derivatives, alkylphenols, (block) polymers, siloxanes, fatty acid salts or mixtures thereof as the emulsifier.

17. Agent according to one of claims 3-7 or 9-16, **characterised in that** it has Abil® EM 90, Arlacel® 582 and magnesium stearate or mixtures thereof as the emulsifier.

18. Agent according to one of claims 3-5 or 8-15, **characterised in that** the emulsifier is chosen from polyoxyethylated products, non-ionic and ionic phosphates, ionic monovalent salts, (poly)glyceryl esters, sugar esters, sterol derivatives, castor oil derivatives, siloxanes or mixtures thereof or mixtures with co-emulsifiers thereof.

19. Agent according to claim 18, **characterised in that** it comprises Tego Care® 450, Eumulgin® B1 or mixtures thereof as the emulsifier.

20. Agent according to one of claims 3-5, 8-16 or 18, 19, **characterised in that** a mixture of acrylamides, acrylates and polysaccharides is used as stabiliser.

21. Agent according to one of claims 1-20, **characterised in that** it has, as additives, those chosen from vitamins, electrolytes, allantoin, D-panthenol, hyaluronic acid, mucopolysaccharides, dyes, perfume substances, preservatives and humectants.

22. Agent according to one of claims 1-21, **characterised in that** it additionally has wax products.

23. Agent according to one of claims 1-22, **characterised in that** it also comprises lecithins as additives.

24. Agent according to one of claims 1-23 for caring for damaged skin tissue.

25. Process for the production of agents according to one of claims 1-23, **characterised in that** the onion extract in which additives soluble therein can be incorporated, and the fat phase are produced, where additives soluble therein can be incorporated, and then the water phase is prepared, which may have additives soluble therein and in particular alcohols, and then the water phase and the fat phase are emulsified at temperatures of 60 to 90°C together with one or more emulsifiers or mixtures thereof or with co-emulsifiers and, after cooling, the additives, if present, are added and processed in a suitable manner, or the onion extract is incorporated together with the additives into the fat (oil) phase, where alcohols may also be added as solvents.

26. Use of an agent according to one of claims 1-23 for producing compositions for treating, caring for or preventing damaged skin tissue.

27. Use according to claim 26, **characterised in that** the damaged skin tissue is scar tissue.

28. Use according to claim 27, **characterised in that** the scar tissue is tissue which has arisen as a result of acne or cosmetic operations.

29. Use according to claim 26, **characterised in that** the damaged skin tissue is stretch marks.

30. Use according to claim 26, **characterised in that** the damaged skin tissue is that which has arisen due to cuts, operation wounds, burns or is damaged skin tissue which has arisen as a result of agerelated degeneration.

## Revendications

1. Agent contenant un extrait d'oignon à usage local, **caractérisé en ce qu'**il contient une phase grasse (huileuse), des additifs choisis parmi des agents de consistance, des colorants, des substances parfumées, des agents humectants, des antioxydants, des conservateurs, des stabilisateurs, des substances actives ajoutées, ainsi qu'un extrait d'oignon.

2. Agent selon la revendication 1, **caractérisé en ce qu'**il présente de 70 à 99% de phase grasse (huileuse), de 0,1 à 20% d'additifs et de 1 à 20% d'extrait d'oignon.

3. Agent selon la revendication 1, **caractérisé en ce qu'**il contient une phase grasse (huileuse), des additifs choisis parmi des agents de consistance, des colorants, des substances parfumées, des agents humectants, des antioxydants, des conservateurs, des stabilisateurs, des substances actives ajoutées et un extrait d'oignon ainsi également qu'une phase aqueuse et un ou plusieurs émulsifiants E/H ou H/E ou leurs mélanges ou des mélanges avec des co-émulsifiants.

4. Agent selon l'une des revendications 1 à 3, **caractérisé en ce que** l'extrait d'oignon est un extrait d'oignon contenant de l'eau, de l'alcool ou de l'eau/alcool.

5. Agent selon la revendication 3 ou la revendication 4, **caractérisé en ce qu'**il contient de 5 à 70% de phase grasse (huileuse), de 0,1 à 15% d'émulsifiants E/H ou H/E ou leurs mélanges ou des mélanges avec des co-émulsifiants, de 1 à 20% d'extrait d'oignon, de 0,1 à 35% d'additifs et pour le reste de l'eau ou un mélange d'eau/alcool.

6. Agent selon l'une des revendications 3 à 5, **caractérisé en ce qu'**il présente une phase grasse (huileuse) en une quantité de 5 à 70%, un ou plusieurs émulsifiants E/H en une quantité totale de 0,5 à 15%, ainsi que leurs mélanges ou des mélanges avec des co-émulsifiants, ainsi que de 0,5 à 25% d'additifs, de 1 à 15% d'extrait d'oignon et pour le reste de l'eau ou un mélange d'eau/alcool.

7. Agent selon la revendication 6, **caractérisé en ce qu'**il présente de 5 à 55% de phase huileuse, de 0,5 à 10% d'un ou plusieurs émulsifiants E/H ou leurs mélanges ou des mélanges avec des co-émulsifiants, de 0,1 à 25% d'additifs, de 1 à 10% d'extrait d'oignon et pour le reste de l'eau ou un mélange d'eau/alcool.

8. Agent selon l'une des revendications 3 à 5, **caractérisé en ce qu'**il présente de 5 à 40% de phase grasse (huileuse), de 0,1 à 15% d'un ou plusieurs émulsifiants H/E ou des mélanges de ceux-ci avec des co-émulsifiants, de 0,1 à 32% d'additifs, de 1 à 10% d'extrait d'oignon et pour le reste de l'eau.

9. Agent selon l'une des revendications 1 à 8, **caractérisé en ce que** l'extrait d'oignon qu'il contient est un extrait à l'éthanol aqueux.

10. Agent selon la revendication 9, **caractérisé en ce que** l'extrait d'oignon contient de l'eau et de 10 à 40% d'éthanol.

11. Agent selon l'une des revendications 1 à 8, **caractérisé en ce que** l'extrait d'oignon qu'il contient est un extrait alcoolique qui présente de 10 à 80% d'un solvant choisi parmi des esters d'acides gras, des triglycérides, des hydrocarbures ou des mélanges de ceux-ci.

12. Agent selon l'une des revendications 1 à 8, **caractérisé en ce que** l'extrait d'oignon est un mélange d'un extrait d'oignon à l'eau/éthanol avec 10 à 40% d'éthanol et d'un extrait alcoolique d'oignon avec 10 à 80% d'un solvant choisi parmi des esters d'acides gras, des triglycérides, des hydrocarbures ou des mélanges de ceux-ci.

13. Agent selon l'une des revendications 1 à 12, **caractérisé en ce que** la phase huileuse (grasse) est choisie parmi des hydrocarbures, des alcools gras, des éthers ou esters d'alcools gras, des esters d'acides gras avec des polyols, des triglycérides, des huiles naturelles, des matières grasses naturelles, des cires, des huiles de silicone, des cires de silicone ou des mélanges de ces substances.

14. Agent selon l'une des revendications 1 à 13, **caractérisé en ce qu'**il présente comme phase huileuse des paraffines liquides, des esters de l'acide lactique, des éthers d'alcools gras, de l'huile de carthame, de l'huile de silicone ou des mélanges de ces substances.

15. Agent selon l'une des revendications 3 à 14, **caractérisé en ce que** l'émulsifiant E/H présente une valeur d'équilibre hydrophile/lipophile HLB de 1 à 8 et **en ce que** l'émulsifiant H/E présente une valeur HLB de 9 à 18 ou est un émulsifiant H/E ionique.

16. Agent selon l'une des revendications 3 à 7 ou 9 à 15, **caractérisé en ce qu'**il contient comme émulsifiant des dérivés de sorbitane, des acides/alcools/esters gras/triglycérides polyéthoxylés, des dérivés de (poly)glycéryle, des esters de polyols, des dérivés de glucose, des dérivés de pentaérythritol, des alkylphénols, des polymères (séquencés), des siloxanes, des sels d'acides gras ou des mélanges de ces substances.

17. Agent selon l'une des revendications 3 à 7 ou 9 à 16, **caractérisé en ce qu'**il présente comme émulsifiant de l'Abil® EM 90, de l'Arlacel® 582 et du stéarate de magnésium ou des mélanges de ces substances.

18. Agent selon l'une des revendications 3 à 5 ou 8 à 15, **caractérisé en ce que** l'émulsifiant est choisi parmi des produits polyéthoxylés, des phosphates non ioniques et ioniques, des sels monovalents ioniques, des esters de (poly)glycéryle, des esters de sucres, des dérivés de stérols, des dérivés de l'huile de ricin, des siloxanes ou leurs mélanges ou des mélanges de ces substances avec des co-émulsifiants.

19. Agent selon la revendication 18, **caractérisé en ce qu'**il contient comme émulsifiant du Tego Care® 450, de l'Eumulgin® B1 ou des mélanges de ceux-ci.

20. Agent selon l'une des revendications 3 à 5, 8 à 16 ou 18, 19, **caractérisé en ce que** le stabilisateur utilisé est un mélange d'acrylamides, d'acrylates et de polysaccharides.

21. Agent selon l'une des revendications 1 à 20, **caractérisé en ce qu'**il présente comme additifs ceux choisis parmi des vitamines, des électrolytes, l'allantoïne, le D-panthénol, l'acide hyaluronique, des mucopolysaccharides, des colorants, des substances parfumées, des conservateurs, des agents humectants.

22. Agent selon l'une des revendications 1 à 21, **caractérisé en ce qu'**il présente en plus des produits à base de cires.

23. Agent selon l'une des revendications 1 à 22, **caractérisé en ce qu'**il contient en plus comme additifs des lécithines.

24. Agent selon l'une des revendications 1 à 23, destiné à soigner les lésions du tissu cutané.

25. Procédé de préparation d'agents selon l'une des revendications 1 à 23, **caractérisé en ce que** l'on prépare l'extrait d'oignon, dans lequel des additifs solubles peuvent être inclus, et la phase grasse, dans laquelle des additifs solubles peuvent être incorporés, et ensuite on prépare la phase aqueuse, qui peut présenter des additifs solubles et notamment des alcools, puis on émulsionne la phase aqueuse et la phase huileuse à des températures dans la plage de 60 à 90°C ensemble avec un ou plusieurs émulsifiants ou mélanges de ceux-ci entre eux ou avec des co-émulsifiants et, après refroidissement, on ajoute les additifs éventuellement présents et on effectue un traitement approprié, ou bien on incorpore l'extrait d'oignon ensemble avec les additifs dans la phase grasse (huileuse), des alcools pouvant également être ajoutés en tant que solvants.

26. Utilisation d'un agent selon l'une des revendications 1 à 23 pour préparer des compositions destinées au traitement, au soin ou à la prévention des lésions du tissu cutané.

27. Utilisation selon la revendication 26, **caractérisée en ce que** la lésion du tissu cutané est un tissu cicatriciel.

28. Utilisation selon la revendication 27, **caractérisé en ce que** le tissu cicatriciel est un tissu résultant de l'acné ou d'interventions de chirurgie esthétique.

29. Utilisation selon la revendication 26, **caractérisé en ce que** les lésions du tissu cutané sont des vergetures de grossesse.

30. Utilisation selon la revendication 26, **caractérisé en ce que** les lésions du tissu cutané sont des lésions du tissu cutané résultant de plaies de coupure, de plaies d'opérations, de brûlures ou d'une dégénérescence liée à l'âge.
